# EUROPEAN PATENT SPECIFICATION

(11) **EP 3 052 938 B1**
(45) Date of publication and mention of the grant of the patent: **05.06.2019**
(21) Application number: 14777634.8
(22) Date of filing: 01.10.2014
(51) Int. Cl.: G01N 33/574

(54) **METHODS AND PRODUCTS FOR THE DIAGNOSIS AND PROGNOSIS OF OVARIAN TUMOR MALIGNANCY**
VERFAHREN UND PRODUKTE ZUR DIAGNOSE UND PROGNOSE VON OVARIALTUMORMALIGNITÄT
PROCÉDÉS ET PRODUITS POUR LE DIAGNOSTIC ET LE PRONOSTIC D'UNE MALIGNITÉ TUMORALE OVARIENNE

(30) Priority: 02.10.2013 ES 201331448
(43) Date of publication of application: 10.08.2016
(73) Proprietor: Oncomatryx Biopharma, S.L., 48160 Derio (Vizcaya) (ES)
(72) Inventor: DOMINGUEZ HORMAETXE, Saioa, 48160 Derio (ES); SIMON BUELA, Laureano, 48160 Derio (ES)
(74) Representative: ABG Intellectual Property, S.L.
(86) International application number: PCT/EP2014/071036
(87) International publication number: WO 2015/049282

(56) References cited:
- WO-A1-2012/142330
- WO-A2-2013/021088
- HOON KIM ET AL: "Multi-cancer computational analysis reveals invasion-associated variant of desmoplastic reaction involving INHBA, THBS2 and COL11A1", BMC MEDICAL GENOMICS, BIOMED CENTRAL LTD, LONDON UK, vol. 3, no. 51, 3 November 2010 (2010-11-03), pages 1-12, XP002713580, ISSN: 1755-8794, DOI: 10.1186/1755-8794-3-51
- Y-H WU ET AL: "COL11A1 promotes tumor progression and predicts poor clinical outcome in ovarian cancer", ONCOGENE, vol. 33, no. 26, 12 August 2013 (2013-08-12), pages 3432-3440, XP055168009, ISSN: 0950-9232, DOI: 10.1038/onc.2013.307
- JAVIER FREIRE ET AL: "Pro-COL11A1: A biomarker to predict malignant relapse of breast intraductal papillomas", JOURNAL OF CLINICAL ONCOLOGY AMERICAN SOCIETY OF CLINICAL ONCOLOGY, vol. 31, no. 2013, 20 March 2013 (2013-03-20), page 540, XP055122151,
- GARCIA-OCANA MARCOS: "Characterization of a novel mouse monoclonal antibody, clone 1E8.33, highly specific for human procollagen 11A1, a tumor-associated stromal component", INTERNATIONAL JOURNAL OF ONCOLOGY, 7 February 2012 (2012-02-07), XP055167934, ISSN: 1019-6439, DOI: 10.3892/ijo.2012.1360
- Carmen Garcã-a-Pravia ET AL: "Overexpression of COL11A1 by Cancer-Associated Fibroblasts: Clinical Relevance of a Stromal Marker in Pancreatic Cancer", PLoS ONE, vol. 8, no. 10, 23 October 2013 (2013-10-23), page e78327, XP55167998, DOI: 10.1371/journal.pone.0078327

## Description

### Field of the Invention

The present invention relates to *in vitro* methods and products for the diagnosis and prognosis of ovarian tumor malignancy.

### Background of the Invention

Cancer is one of the main health problems in the world. According to the GLOBOCAN database of the International Agency for Research on Cancer of the World Health Organization, more than 12 million cancer cases were diagnosed worldwide in 2008, and the number of deaths due to cancer in 2008 was more than 7 million people.

During the process of malignization, tumor cells change their protein expression pattern, which impairs cellular processes, such as the maintenance of cell architecture, cell proliferation, adhesion and death. These modifications do not only generate changes in the tumor cells themselves, but also cause some cells in the stroma surrounding the tumor to receive impaired molecular signals and change their pattern of behavior and protein expression pattern, acquiring a morphology typical of myofibroblasts. The molecules secreted by these myofibroblasts in response to adjacent tumor can in turn contribute to promoting tumor growth and invasion in a different way, such that a paracrine loop is established between the tumor and stroma. In recent years, scientific evidence pointing with increasing precision to peritumoral stroma as one of the main promoters of tumor invasiveness, as well as of the phenomena of therapy resistance, have been generated.

The methods and products for the diagnosis and prognosis of ovarian tumor malignancy object of the present invention are encompassed in this novel research framework, since they are based on the use of proCOL11A1 protein present in the stromal cells of malignant carcinomas as an ovarian tumor diagnosis and malignancy marker.

Ovarian cancer is the gynecological disease causing the highest number of deaths in developed countries. With an incidence of 204,000 cases a year, it causes 125,000 deaths worldwide. The 5-year mortality is about 70-80%, in most cases being due to tumor progression and metastasis.

The most common type of ovarian cancer is ovarian epithelial cancer (90%). Epithelial tumors include benign tumors, low malignant potential tumors or borderline tumors and malignant tumors, the prognosis of which depends fundamentally on the stage (I-III) (Ovarian Cancer Detailed Guideline, ACS 2013). Like in other tumors, surgical and pharmacological treatment suitable for each type of tumor depends on the spread of the tumor and on its risk of progression; but in ovarian cancer surgery decision must be the most conservative possible in order to prevent loss of fertility in patients. For this reason, it is of vital importance to correctly classify the tumors and evaluate the tumor progression and metastasis capacity at the time of diagnosis, which would allow selecting the correct surgical procedure for each patient.

Low malignant potential tumors or borderline tumors are benign tumors which can progress into malignant and invasive tumors. These tumors tend to spread outside the ovaries generating implants which can be invasive or non-invasive. Attempt has been made to associate the invasiveness of these implants with the risk of malignization of borderline tumors, but it is very difficult to evaluate the same, particularly in biopsy samples and there are no criteria today that allow classifying borderline tumors according to their progression capacity. Furthermore, these tumors may be mistaken for stage I malignant tumors, given that they share many morphological characteristics. These low malignant potential tumors, as well as benign tumors, are often present together with malignant tumors and can mask said tumors, making the detection thereof difficult, particularly in small biopsies.

Due to the foregoing, a diagnostic system which allows accurately distinguishing benign lesions from malignant ovarian tumors, as well as predicting the risk of malignization of borderline tumors, allowing selecting the most suitable treatment for each patient, assuring disease elimination and preventing excessive or unnecessary treatments which may interfere with patient fertility, is necessary.

Collagen is the main component of the extracellular matrix (ECM). The correct expression of the genes encoding the different types of collagen is necessary for correct ECM assembly during embryonic development and for the maintenance thereof in the adult body. Collagen XI (COL11) is a little-studied type of collagen which, however, plays a fundamental role in the regulation of fibrillar networks in cartilaginous and non-cartilaginous matrices; these fiber networks are involved in different morphogenesis processes during embryonic development in vertebrate animals. Transcripts of the alpha chain 1 (α1) of collagen XI (COL11A1) have been found during fetal development in cartilaginous tissues and also in other tissues such as the bone, kidney, skin, muscle, tongue, intestine, liver, ear, brain and lung. The extracellular matrix also has an important role in specific biological processes, such as cell differentiation, proliferation and migration; therefore, the deregulation of the expression of genes encoding the proteins forming same, is associated with carcinogenesis and metastasis processes. In the particular case of COL11A1, it has been demonstrated that stromal fibroblasts have high expression levels of the *col11a1* gene in sporadic colorectal carcinomas, whereas this gene is not expressed in healthy colon. The expression of the *col11a1* gene has also been associated with pancreatic cancer, breast cancer, colon cancer, lung cancer, head and neck cancer and bladder cancer and the expression of the COL11A1 protein has been associated with pancreatic cancer and colon cancer.

Recent studies have detected high expression of messenger RNA (mRNA) transcript of the *col11a1* gene in advanced stage ovarian tumors (Kim, H et al. BMC Med Genomics. 2010 Nov 3;3:51), recurrent ovarian tumors or ovarian tumors with a poor prognosis (Wu, YH et al; Oncogene. 2013 Aug 12), suggesting the possible role of this molecule in tumor progression, although the presence of the protein it encodes, COL11A1, or the precursor protein thereof, proCOL11A1 protein, in tumors of patients with ovarian cancer has yet to be described. The high expression of the mRNA transcript of a gene does not necessarily lead to the high expression of the protein encoded by this gene (Gigy et al., Mol. Cel. Biol., 1999, 9:1720-1733; Greenbaum et al., Genome Biology, 2003, 4:117). WO2013/021088A2 discloses a method for detecting invasive carcinoma in an individual using antibody 1E8.33 which specifically recognizes proCOL11A1 protein. WO2012/142330A1 relates to *in vitro* methods for detecting the presence of an invasive carcinoma for determining and/or predicting the stage and/or invasiveness of the carcinoma or for monitoring the effect of the therapy based on col11a1 gene and proCOL11A1 protein expression. Hoon Kim et al (BMC, Medical genomics, November 2011, vol. 3, no 51) and Y-H Wu et al (Oncogene, August 2013, vol. 33, no. 26) identified the overexpression of COLL11A1 as an indicator of ovarian cancer stage and invasiveness. Javier Freire et al (Journal of Clinical Oncology (2013, ASCO Annual Meeting Abstracts, vol. 31, no 15) and Garcia-Ocana Marcos et al (International Journal of Oncology, February 2012, XP055167934) disclose proCOL11A1 as biomarker for cancer other than ovarian cancer.

The authors of the present invention have developed methods and products for detecting the proCOL11A1 protein in ovarian tumors and accurately distinguishing benign lesions from malignant ovarian tumors, as well as making a prognosis of the risk of malignization of borderline tumors.

### Brief Description of the Invention

The authors of the present invention have discovered, by means of immunohistochemical analysis of ovarian tissue biopsies using the monoclonal antibody 1E8.33 described in International Patent Application WO 2013/021088 A2, that the proCOL11A1 protein is expressed in malignant ovarian tumors (carcinomas), but is not expressed in non-tumoral ovarian tissue, or in *in situ* or benign or borderline ovarian tumors.

This evidence turns proCOL11A1-specific antibodies into ideal antibodies for developing new *in vitro* methods and products for the diagnosis of ovarian cancer as well as for differentiating malignant ovarian tumors from benign or *in situ* tumors in diagnostic biopsy, or in surgical specimens, or in fluids from the patient, including but not being limited to, blood, serum, plasma, ascites fluid or urine, for making a prognosis of ovarian tumor malignancy and for predicting borderline tumor malignancy, therefore guiding the surgery decision and follow-up. Specifically, the invention provides methods and products for identifying, with high sensitivity and specificity, malignant ovarian tumors *in vitro,* even in the cases in which the morphology of the tumor and the current markers are not detected.

In one aspect, the invention relates to an *in vitro* method for the differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion in a subject, selected from Method (A) and Method (B), wherein
A) Method (A) comprises:
   - detecting the presence of proCOL11A1 protein in a sample obtained by means of biopsy, cytology or surgical resection from said subject;
   wherein
   - the detection of the presence of proCOL11A1 protein in said sample is indicative that the ovarian tumor is malignant; or, alternatively
   - the non-detection of the presence of proCOL11A1 protein in said sample is indicative that the ovarian tumor is benign or *in situ,* or that the subject has a benign non-tumoral ovarian lesion; and
B) Method (B) comprises:
   - comparing the expression level of the proCOL11A1 protein in said sample with the expression level of said proCOL11A1 protein in a control sample;
   wherein
   - an expression level of the proCOL11A1 protein in said sample greater than the expression level of said proCOL11A1 protein in a control sample is indicative that the ovarian tumor is a malignant tumor, or, alternatively
   - an expression level of the proCOL11A1 protein in said sample equal to or less than the expression level of said proCOL11A1 protein in a control sample is indicative that the ovarian tumor is benign or *in situ,* or that the subject has a benign non-tumoral ovarian lesion.

In another aspect, the invention relates to an *in vitro* method for the diagnosis or prognosis of ovarian tumor malignancy in a subject, wherein said subject is a subject suspected of having a malignant ovarian tumor, wherein said malignant ovarian tumor is a malignant tumor in ovaries which can be serous, mucinous, endometrioid, clear cell tumor, or undifferentiated tumor, or is a subject diagnosed with a low malignant potential or borderline ovarian tumor, with or without extraovarian implantations, selected from Method (A) and Method (B), wherein
A) Method (A) comprises
   - detecting the presence of proCOL11A1 protein in a sample obtained by means of biopsy, cytology or surgical resection, from said subject suspected of having a malignant ovarian tumor or diagnosed with a low malignant potential or borderline ovarian tumor, with or without extraovarian implantations;
   wherein
   - the detection of the presence of proCOL11A1 protein in said sample from said subject suspected of having a malignant ovarian tumor or diagnosed with a low malignant potential or borderline ovarian tumor, with or without extraovarian implantations, is indicative that said subject has a malignant ovarian tumor or that said low malignant potential or borderline tumor, with or without extraovarian implantations, is capable of progressing into a malignant ovarian tumor, or, alternatively
   - the non-detection of the presence of proCOL11A1 protein in said sample from said subject suspected of having a malignant ovarian tumor or diagnosed with a low malignant potential or borderline ovarian tumor, with or without extraovarian implantations, is indicative that said subject does not have a malignant ovarian tumor, or that said subject has a benign or *in situ* ovarian tumor, or that said low malignant potential or borderline tumor is not capable of progressing into a malignant ovarian tumor; and
B) Method (B) comprises
   - comparing the expression level of the proCOL11A1 protein in said sample from said subject suspected of having a malignant ovarian tumor or diagnosed with a low malignant potential or borderline ovarian tumor, with or without extraovarian implantations, with the expression level of said proCOL11A1 protein in a control sample;
   wherein
   - an expression level of the proCOL11A1 protein in said sample from said subject suspected of having a malignant ovarian tumor or diagnosed with a low malignant potential or borderline ovarian tumor, with or without extraovarian implantations, greater than the expression level of said proCOL11A1 protein in a control sample is indicative that said subject has a malignant ovarian tumor or that said low malignant potential or borderline tumor is capable of progressing into a malignant ovarian tumor, or, alternatively
   - an expression level of the proCOL11A1 protein in said sample from said subject suspected of having a malignant ovarian tumor or diagnosed with a low malignant potential or borderline ovarian tumor, with or without extraovarian implantations, equal to or less than the expression level of said proCOL11A1 protein in a control sample is indicative that the subject does not have a malignant ovarian tumor, or that said subject has a benign or *in situ* ovarian tumor, or that said low malignant potential or borderline tumor is not capable of progressing into a malignant ovarian tumor.

In another aspect, the invention relates to an *in vitro* method for determining the malignization probability of a low malignant potential or borderline ovarian tumor, with or without extraovarian implantations, in a subject diagnosed with said tumor, selected from Method (A) and Method (B), wherein
A) Method (A) comprises
   - detecting the presence of proCOL11A1 protein in a sample obtained by means of biopsy, cytology or surgical resection from said subject diagnosed with a low malignant potential or borderline ovarian tumor, with or without extraovarian implantations;
   wherein
   - the detection of the presence of proCOL11A1 protein in said sample from said subject diagnosed with a low malignant potential or borderline ovarian tumor, with or without extraovarian implantations, is indicative that the probability of the tumor progressing into a malignant ovarian tumor is high, or, alternatively
   - the non-detection of the presence of proCOL11A1 protein in said sample from said subject diagnosed with a low malignant potential or borderline ovarian tumor, with or without extraovarian implantations, is indicative that the probability of the tumor progressing into a malignant ovarian tumor is low; and
B) Method (B) comprises
   - comparing the expression level of the proCOL11A1 protein in said sample from said subject diagnosed with a low malignant potential or borderline ovarian tumor, with or without extraovarian implantations, with the expression level of said proCOL11A1 protein in a control sample;
      wherein
   - an expression level of the proCOL11A1 protein in said sample from said subject diagnosed with a low malignant potential or borderline ovarian tumor, with or without extraovarian implantations, greater than the expression level of said proCOL11A1 protein in a control sample is indicative that the probability of the tumor progressing into a malignant ovarian tumor is high; or, alternatively
   - an expression level of the proCOL11A1 protein in said sample from said subject diagnosed with a low malignant potential or borderline ovarian tumor, with or without extraovarian implantations, equal to or less than the expression level of said proCOL11A1 protein in a control sample is indicative that the probability of the tumor progressing into a malignant ovarian tumor is low.

In another aspect, the invention relates to a method for selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment, , or for selecting a subject diagnosed with a low malignant potential or borderline ovarian tumor, with or without extraovarian implantations, for treatment, wherein said treatment comprises surgically removing said ovarian tumor, performing additional analyses to evaluate tumor infiltration and metastasis if the subject is suspected of having a malignant ovarian tumor, choosing a chemotherapy and/or radiotherapy treatment, and the subsequent follow-up, said method comprising:
- detecting the presence of proCOL11A1 protein in a sample obtained by means of biopsy, cytology or surgical resection from said subject suspected of having the malignant ovarian tumor, or from said subject diagnosed with a low malignant potential or borderline ovarian tumor, with or without extraovarian implantations; or
- comparing the expression level of the proCOL11A1 protein in said sample from said subject suspected of having a malignant ovarian tumor, or in said sample from said subject diagnosed with a low malignant potential or borderline ovarian tumor, with or without extraovarian implantations, with the expression level of said proCOL11A1 protein in a control sample;
wherein said subject is selected for said treatment:
- if the presence of proCOL11A1 protein is detected in said sample from the subject suspected of having a malignant ovarian tumor, or in said sample from said subject diagnosed with a low malignant potential or borderline ovarian tumor, with or without extraovarian implantations;; or
- if the expression level of the proCOL11A1 protein in said sample from said subject suspected of having a malignant ovarian tumor, or in said sample from said subject diagnosed with a low malignant potential or borderline ovarian tumor, with or without extraovarian implantations, is greater than the expression level of said proCOL11A1 protein in the control sample;
or, alternatively
wherein said subject is not selected for said treatment:
- if the presence of proCOL11A1 protein is not detected in said sample from said subject suspected of having a malignant ovarian tumor, or in said sample from said subject diagnosed with a low malignant potential or borderline ovarian tumor, with or without extraovarian implantations; or
- if the expression level of the proCOL11A1 protein in said sample from said subject suspected of having a malignant ovarian tumor, or in said sample from said subject diagnosed with a low malignant potential or borderline ovarian tumor, with or without extraovarian implantations, is equal to or less than the expression level of said proCOL11A1 protein in the control sample.

In another aspect, the invention relates to a method for selecting a subject diagnosed with a low malignant potential or borderline ovarian tumor, with or without extraovarian implantations, for follow-up, wherein said follow-up comprises surgically resectioning said tumor and monitoring tumor recurrence, said method comprising:
- detecting the presence of proCOL11A1 protein in a sample obtained by means of biopsy, cytology or surgical resection from said subject diagnosed with a low malignant potential or borderline ovarian tumor, with or without extraovarian implantations; or
- comparing the expression level of the proCOL11A1 protein in said sample from said subject diagnosed with a low malignant potential or borderline ovarian tumor, with or without extraovarian implantations, with the expression level of said proCOL11A1 protein in a control sample;
wherein said subject is selected for said follow-up:
- if the presence of proCOL11A1 protein is detected in said sample from the subject diagnosed with a low malignant potential or borderline ovarian tumor, with or without extraovarian implantations; or
- if the expression level of the proCOL11A1 protein in said sample from said subject diagnosed with a low malignant potential or borderline ovarian tumor, with or without extraovarian implantations, is greater than the expression level of said proCOL11A1 protein in the control sample.

In another aspect, the invention relates to the use of the proCOL11A1 protein as a marker for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor,, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

In another aspect, the invention relates to the *in vitro* use of a specific antibody recognizing the proCOL11A1 protein for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

In another aspect, the invention relates to the *in vitro* use of a kit comprising a reagent recognizing the proCOL11A1 protein, or a reagent for the detection and/or quantification of the expression of the proCOL11A1 protein, for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

### Brief Description of the Drawings

Figure 1 shows the results of the immunostain for proCOL11A1 in a sample of a benign ovarian tumor (A), a serous malignant ovarian tumor (carcinoma) (B) and the metastasis of a colon tumor into an ovarian tumor (C).
Figure 2 is a summary table showing the number of cases classified according to the diagnosis of the biopsy and according to the immunolabeling for proCOL11A1. Additional information can be found in Example 1.

### Detailed Description of the Invention

### Definitions

To make it easier to understand the present patent application, the meaning of some terms and expressions as they are used within the context of the present invention is provided below.

The term "antibody" refers to a glucoprotein exhibiting a specific binding activity with a particular protein referred to as an "antigen". The term "antibody" comprises whole monoclonal antibodies or polyclonal antibodies, or fragments thereof; and includes human antibodies, humanized antibodies and antibodies of non-human origin. "Monoclonal antibodies" are homogeneous populations of highly specific antibodies targeting a unique site or antigenic "determinant".

The term "cancer" or "carcinoma" refers to the disease which is characterized by an uncontrolled proliferation of abnormal cells capable of invading adjacent tissues and spreading to distant organs.

The term "ovarian cancer", "ovarian carcinoma" or "ovarian tumor" refers to any proliferative disorder of ovary cells, being able to be benign, malignant, *in situ,* or low malignant potential or borderline, being specified in each case.

As it is used herein, the term "capable of malignization" or "capable of progressing into a malignant tumor" refers to a benign tumor or a low malignant potential tumor being capable of becoming a malignant tumor.

As it is used herein, the term "diagnosis" applied to a malignant ovarian tumor, or to a low malignant potential or borderline ovarian tumor being capable of progressing into a malignant tumor, includes determining the malignancy of said ovarian tumors or whether or not the ovarian tumors are capable of malignization.

As it is used herein, the term "epitope" refers to an antigenic determinant of a protein which is the amino acid sequence of the protein recognized by a specific antibody.

The term "specificity" refers to the capacity to detect true negatives. A specificity of 100% means that there are no false positive (non-affected subjects that obtain positive result).

The term "infiltration" refers to the presence of tumor cells outside the primary tumor due to tumor invasion.

The term "gene" refers to a molecular deoxyribonucleotide chain encoding a protein.

The term "col11a1 gene" refers to the gene encoding a collagen XI component referred to as "pro-α1(XI) chain" which combines with other two collagen chains (pro-α2(XI) and pro-α1(II)) to form a procollagen molecule (proCOL11A1) which is enzymatically processed in cells to form collagen XI fibers. Human col11a1 gene (also referred to as COLL6 or STL2), the reference gene sequence of which is NG_008033.1, spans over 150 kilobases (kb), contains 68 exons, is located in chromosome 1 (1p21) between the base pairs 103342023 and 103574052, and encodes a protein of 1,806 amino acids (according to the isoform) and 181 KDa containing a signal peptide (amino acids 1-36). This gene is conserved in humans, chimpanzee, cow, chicken, mouse, rat and zebrafish. Mutations in this gene have been associated with Stickler syndrome type II and Marshall syndrome. Polymorphisms of a nucleotide in this gene have been associated with the susceptibility of having spinal disc herniation. Several transcripts differing from one another mainly in the transcription of the variants of exon 6 have been described, for example, a transcript of 7.2 kb encoding isoform A (NM_001854.3 (GI:98985806), NCBI database as of July 21, 2011) of 1,806 amino acids, another transcript of 7,3 kb encoding isoform B (NM_080629.2 NCBI database as of July 21, 2011) of 1,806 amino acids (GI:98985810), another transcript of 6.9 kb encoding isoform C (NM_080630.3 (GI:299523252), NCBI database as of July 21, 2011) of 1,690 amino acids (GI:299523253), another transcript of 7.2 kb encoding isoform E (NM_001190709.1, NCBI database as of August 1, 2011) of 1,767 amino acids (GI:299523257), etc. As it is used herein, the term "*col11a1*" does not refer only to the human gene but also to the orthologs of other species. Low and homogeneous expression of the *col11a1* gene is observed in all analyzed tissues except in adipocytes where it is significantly higher (BioGPS: Gene Atlas U133A). In cell lines, high expression is detected in UASMC cells (umbilical artery smooth muscle cells), HN NP cells (neuronal precursors), HN_Os (osteoblasts), Panc-1 (pancreatic cancer cells), H522 (lung cancer cells), U251 (glioma cells) (RefExA), A-204 (heart rhabdomyosarcoma cells), SAOS-2 (bone marrow osteosarcoma cells) and SK-MEL28 (skin cancer cells) (GeneCards).

The terms "equal" or "less" applied to the expression level of a protein, specifically the proCOL11A protein, mean that the amount or concentration of said protein in a specific sample is substantially the same as (equal to), or lower than (less than), that found in another sample considered as a control sample or a reference value; therefore the expression level of the proCOL11A1 protein in a sample from a subject suspected of having a malignant ovarian tumor or diagnosed with a low malignant potential or borderline tumor, is equal (substantially the same) to or less (lower) than the expression level of said proCOL11A1 protein in a control sample obtained from a control population of subjects without history of ovarian tumors. In the context of the present invention, the expression level of a protein, such as the proCOL11A1 protein, in the sample from the subject suspected of having a malignant ovarian tumor or diagnosed with a low malignant potential or borderline ovarian tumor, is considered "equal" to the expression level of said protein in the control sample when the expression level of said protein in the sample from the subject is substantially the same as the expression level of said protein in the control sample, i.e., when it is comprised, for example, between the reference value (expression level of the proCOL11A1 protein) plus/minus an amount lower than 3% of said reference value. Likewise, in the context of the present invention, the expression level of a protein, such as the proCOL11A1 protein, in the sample from the subject suspected of having a malignant ovarian tumor, or diagnosed with a low malignant potential or borderline ovarian tumor, is considered "less" (or "lower") than the expression level of said protein in the control sample when the expression level of said protein in the sample from the subject decreases, for example, by 3%, 5%, 10%, 25%, 50%, or even 100%, with respect to the reference value.

As it is used herein, the term "malignancy" refers to tumor capacity to spread to or invade the tissue in which it is located, the adjacent or distal tissues, being life-threatening to the subject having the tumor.

As it is used herein, the term "probability" measures the frequency whereby a result (or a group of results) is obtained when carrying out a random experiment, from which all the possible results are known, under sufficiently stable conditions. The probability that a specific result is detected can be "high", i.e., the frequency whereby a result is obtained is greater than 50%, or "low", i.e., the frequency whereby such result is obtained is less than 50%. According to the present invention, the probability that the proCOL11A1 protein is detected in a sample from a subject suspected of having a malignant ovarian tumor or diagnosed with a low malignant potential or borderline ovarian tumor, or the probability that the expression level of the proCOL11A1 protein in a sample from a subject suspected of having a malignant ovarian tumor or diagnosed with a low malignant potential or borderline ovarian tumor is greater than the expression level of the proCOL11A1 protein in a control sample, is higher in those cases in which said subject has a malignant ovarian tumor, or in which said low malignant potential or borderline tumor is capable of progressing into a malignant tumor. As will be understood by the persons skilled in the art, the probability does not have to be 100% for all the evaluated subjects, although it must preferably be so. However, within the context of the present invention, said term requires being able to identify a statistically significant part of the subjects suspected of having a malignant ovarian tumor or diagnosed with a low malignant potential or borderline ovarian tumor as subjects having a higher probability of obtaining a specific result. The person skilled in the art can determine whether or not an event is statistically significant without major complications using different known statistical evaluation tools, for example, by means of determining the confidence intervals, determining the p-value, the Student's t-test, the Mann-Whitney test, etc. Additional information about these statistical tools can be found in Dowdy and Wearden, Statistics for Research. John Wiley & Sons, New York, 1983. The preferred confidence intervals are at least 50%, at least 60%, at least 70%, at least 80%, at least 90%, or at least 95%. The p-values are preferably 0.1, 0.05, 0.02, 0.01 or less.

The term "proCOL11A1" refers to the precursor of collagen XI α1 chain, the processing of which gives rise to collagen XI α1 (COL11A1). proCOL11A1 has a central triple-helix domain with a rod-shaped structure, which is flanked by non-collagenous propeptides at the N- and C-terminal ends; these propeptides can be eliminated by specific peptidases as they are secreted out of the cell generating mature COL11A1, where collagen XI α1 chains are assembled with one another and with α2 and/or α3 chains to form collagen XI fibers. proCOL11A1 protein is encoded by the *col11a1* gene. Several isoforms of human proCOL11A1 have been described, for example, isoform A [NM_001854.3 (GI:98985806), NCBI database as of July 21, 2011] of 1,806 amino acids, isoform B [NP_542196.2 (GI:98985810), NCBI database as of July 21, 2011], isoform C [NM_080630.3 (GI:299523252), NCBI database as of July 21, 2011], etc. As it is used herein, the term "proCOL11A1" does not refer only to human proCOL11A1 protein but also to the orthologs of other species.

The term "protein" refers to a molecular chain of amino acids with biological activity. The term includes all forms of post-translation modifications, for example, glycosylation, phosphorylation or acetylation. The terms "protein", "peptide" and "polypeptide" are used interchangeably throughout this description.

As it is used herein, the term "follow-up" refers to the group of measures to be applied and assessed once the nature of an ovarian tumor in a subject is diagnosed and once a decision on whether or not to use surgical approach on the tumor is made; said measures include, among others, depending on the nature of the ovarian tumor, analyzing the spread of the tumor outside the ovary including the location of possible points of metastasis, applying surgical or pharmacological treatment suitable for the diagnosis of said tests, performing periodic analyses along with or after treatment, which allow detecting tumor relapses, etc. Therefore, the term "follow-up" applied to a subject suspected of having a malignant ovarian tumor who is verified as having a malignant ovarian tumor (either because the presence of proCOL11A1 protein is detected in a sample from said subject, or because the expression level of the proCOL11A1 protein in a sample from said subject is greater than the expression level of proCOL11A1 in a control sample), and from whom the tumor has been eliminated by means of surgery, refers to the analysis of the spread of the tumor outside the ovary by means of lymph nodes sampling to search for tumor presence, image analysis by suitable techniques such as ultrasound or CAT scan to search for the points of metastasis, application of surgical or pharmacological treatment suitable for the diagnosis of said tests, and the performance of periodic analyses along with or after treatment, which allow detecting tumor relapses, for example, by means of using imaging techniques or analyzing serum markers, such as tumor marker CA-125 (also referred to as "mucin 16" or "MUC16"), for example. Likewise, the term "follow-up" applied to a subject with a low malignant potential or borderline ovarian tumor, with or without extraovarian implantations, diagnosed with a tumor with the risk of malignization (either because the presence of proCOL11A1 protein is detected in a sample from said subject, or because the expression level of the proCOL11A1 protein in a sample from said subject is greater than the expression level of proCOL11A1 in a control sample), and, optionally, if the specialist deemed it suitable, subjected to a surgical resection of the tumor, includes monitoring possible tumor recurrence by means of performing periodic tests by imaging techniques or by analyzing serum markers, such as CA125. In this case, surgical resection of the tumor is carried out before monitoring possible tumor recurrence by means of performing periodic tests by imaging techniques or by analyzing serum markers, such as CA-125.

The term "sensitivity" refers to the detection of true positives (for example, positive diagnosis of a pathology when the patient affected by said pathology); 100% sensitivity means that there are no false negatives (negative diagnosis in affected patients).

The term "subject", "individual" or "patient" refers to a member of a mammalian species and includes, but is not limited to, domestic animals, primates and humans; the subject is preferably a male or female human being of any age or race.

The term "greater" applied to the expression level of a protein, specifically the proCOL11A protein, means that the amount or concentration of said protein in a specific sample is higher than in another sample considered as a control sample or a reference value; therefore the expression level of the proCOL11A1 protein in a sample from a subject suspected of having a malignant ovarian tumor or diagnosed with a low malignant potential or borderline ovarian tumor, is higher (greater) than the expression level of said proCOL11A1 protein in a control sample obtained from a control population of subjects without history of ovarian tumors, in case that said subject has a malignant ovarian tumor or in case that said low malignant potential or borderline tumor is capable of progressing into a malignant tumor. In the context of the present invention, the expression level of a protein, such as the proCOL11A1 protein, in the sample from the subject suspected of having a malignant ovarian tumor or diagnosed with a low malignant potential or borderline ovarian tumor is considered "greater" (or "higher") than the expression level of said protein in a control sample (reference value) when the expression level of said protein in the sample from the subject increases, for example, by 3%, 5%, 10%, 25%, 50%, 100%, or even more, compared with the reference value for said protein in the control sample, or when it increases, for example, at least by 1.1-fold, 1.5-fold, 2-fold, 5-fold, 10-fold, 20-fold, 30-fold, 40-fold, 50-fold, 60-fold, 70-fold, 80-fold, 90-fold, 100-fold, or even more, when compared with the reference value for said protein.

As it is used herein, the term "treatment" generally refers to the application of a therapy to alleviate or eliminate a pathology or to reduce or eliminate one or more symptoms associated with said pathology. Said therapy can include a surgical intervention, a pharmacological treatment, a radiotherapy treatment, etc.

The term "tumor" refers to any abnormal tissue mass resulting from a benign (non-cancerous) or malignant (cancerous) neoplastic process. A benign tumor is a tumor that is not capable of spreading to or invading neither the tissue in which it is located nor the adjacent or distal tissues, such that it is not life-threatening to the subject having the tumor. In contrast, a malignant tumor is a tumor capable of spreading to or invading the tissue in which it is located, the adjacent or distal tissues, such that it is life-threatening to the subject having the tumor. Malignant tumors in ovaries can be serous, mucinous, endometrioid or clear cell tumor, or undifferentiated tumor. Likewise, a low malignant potential or borderline ovarian tumor is an ovarian tumor which, while not being benign, has no clear malignancy characteristics, but can progress in some cases into malignant tumor and grow and spread throughout the abdominal cavity.

As it is used herein, the term "variant" applied to the heavy and light chain sequences of an antibody refers to substantially similar sequences. Generally, from the qualitative viewpoint, variants have the same biological activity as the native sequence. A variant of a polypeptide sequence can be a derivative of a polypeptide sequence comprising addition, deletion or substitution of one or more amino acids present in the native sequence. The variants of the heavy and light chain sequences of an antibody can differ from the sequences described within the frame regions or within the complementarity determining regions or CDRs of any of the heavy or light chains. By way of illustration, the term "variant" applied to the monoclonal antibody 1E8.33 (García-Ocaña, et al., Int J Oncol. 2012; 40(5):1447-54) includes amino acid sequences at least having about at least 70%, 75%, 80%, 85%, 90%, 91%, 92%, 93%, 94%, 95%, 96%, 97%, 98% or 99% of sequence identity with any of the amino acid sequences of the CDRs comprised within the variable region of the heavy chain or the variable region of the light chain of said monoclonal antibody 1E8.33, provided that the resulting variant maintains the biological activity of the native sequence, i.e., the variant of the antibody maintains the capacity of specifically recognizing the proCOL11A1 protein.

### Methods of the Invention

The present invention is based on the discovery that the expression of proCOL11A1 protein increases in malignant ovarian tumors or in tumors capable of progressing into a malignant tumor, but not in *in situ* tumors, benign tumors, low malignant potential or borderline tumors that are not capable of progressing into a malignant tumor, or in benign lesions. The expression of proCOL11A1 occurs in fibroblasts and in the extracellular matrix surrounding the tumor, unlike other tumor markers which are expressed in the cancerous cells. Therefore, diagnosis based on proCOL11A1 allows detecting malignant ovarian tumors although the infiltration is not morphologically obvious in the primary tumor present in the biopsy, and allows assessing if a low malignant potential or borderline ovarian tumor are capable of malignization at the time of diagnosis.

### 1. Method for the differential diagnosis of a malignant ovarian tumor from a benign or in situ ovarian tumor or from a benign non-tumoral ovarian lesion

In another aspect, the invention relates to an *in vitro* method for the differential diagnosis of a malignant ovarian tumor from a benign or *in situ* ovarian tumor or from a benign non-tumoral ovarian lesion in a subject, hereinafter "first method of the invention", selected from Method (A) and Method (B), wherein
A) Method (A) comprises:
   - detecting the presence of proCOL11A1 protein in a sample from said subject;
   wherein
   - the detection of the presence of proCOL11A1 protein in said sample is indicative that the ovarian tumor is malignant; or, alternatively
   - the non-detection of the presence of proCOL11A1 protein in said sample is indicative that the ovarian tumor is a benign or *in situ* tumor, or that the subject has a benign non-tumoral ovarian lesion; and
B) Method (B) comprises:
   - comparing the expression level of the proCOL11A1 protein in said sample with the expression level of said proCOL11A1 protein in a control sample;
   wherein
   - an expression level of the proCOL11A1 protein in said sample greater than the expression level of said proCOL11A1 protein in a control sample is indicative that the ovarian tumor is a malignant tumor, or, alternatively
   - an expression level of the proCOL11A1 protein in said sample equal to or less than the expression level of said proCOL11A1 protein in a control sample is indicative that the ovarian tumor is a benign or *in situ* tumor, or that the subject has a benign non-tumoral ovarian lesion.

Therefore according to the first method of the invention, the presence of proCOL11A1 protein is detected and/or the expression level of said proCOL11A1 protein is determined in a sample from the subject under study and the obtained result is correlated with possible ovarian tumor malignancy or with the existence of a benign non-tumoral ovarian lesion.

The subject under study can be a subject having a tumoral or non-tumoral lesion in the ovary, such as, for example, a subject with an ovarian tumor, a subject suspected of having a ovarian tumor, a subject suspected of having a malignant ovarian tumor, a subject diagnosed with a low malignant potential or borderline ovarian tumor, a subject with a benign ovarian tumor, a subject with an *in situ* ovarian tumor, or a subject with a benign non-tumoral ovarian lesion.

For putting the first method of the invention into practice in any of the alternatives thereof [Method (A) or (Method (B)], a sample, such as a biological sample, is obtained from the individual to be studied. Non-limiting illustrative examples of said sample include a core of tissue obtained by means of biopsy, a tissue specimen obtained by surgery, as well as a biological fluid, such as blood, serum, ascites fluid, urine, etc. In a particular embodiment, said sample comprises tissue obtained by means of biopsy or surgery; in a more particular embodiment, said sample comprising tissue comprises tumor-associated stromal cells, preferably, cancer-associated fibroblasts (also referred to as CAF), expressing proCOL11A1. In another particular embodiment, said sample comprises a biological fluid (e.g., blood, serum, ascites fluid, urine, etc.). Depending on the type of samples, to simplify their preservation and handling, these can be fixed in formalin and embedded in paraffin, or, alternatively, they can be first frozen and then embedded in a medium that can be cryogenically solidified.

The samples to be analyzed can be obtained from subjects after an ovarian tumor has been diagnosed, or has not been diagnosed, treated or non-treated.

### Method (A)

In a particular embodiment, the first method of the invention comprises detecting the presence of proCOL11A1 protein in a sample obtained by means of biopsy, cytology or surgical resection from said subject [Method (A)], wherein the detection of the presence of proCOL11A1 protein in said sample is indicative that the ovarian tumor (or carcinoma) is malignant; or alternatively the non-detection of the presence of proCOL11A1 protein in said sample is indicative that the ovarian tumor is benign or in situ, or that the subject has a benign non-tumoral ovarian lesion.

The presence of proCOL11A1 protein in the sample to be analyzed can be detected by means of any conventional method which allows detecting the presence of a protein, specifically proCOL11A1, in a sample. In a particular embodiment, said sample is an ovarian tissue sample or abdominal cavity sample or a body fluid sample. Virtually any conventional method for detecting the presence of a protein in a tissue sample or a body fluid sample can be used within the scope of the invention for detecting the presence of proCOL11A1. Non-limiting illustrative examples of said methods include methods based on the use of antibodies, affinity chromatography techniques, ligand binding assays, etc.

In a particular embodiment, the detection of the presence of proCOL11A1 protein is performed by means of an immunoassay based on the formation of an antigen-antibody type complex by means of using one (or more) antibodies recognizing one (or more) epitopes of proCOL11A1, and the viewing the complexes formed by any suitable technique, including both radioctive and non-radioctive techniques such as, for example, colorimetric techniques, fluorometric techniques, luminiscent (e.g., bioluminiscent, chemiluminiscent, etc.) techniques, etc., using in turn secondary antibodies labeled with the suitable markers to that end.

The antibodies to be used for putting this particular embodiment of the first method of the invention [Method (A)] into practice are specific antibodies recognizing the proCOL11A1 protein, or an antigenic fragment of said proCOL11A1 protein, i.e., antibodies recognizing an epitope of the proCOL11A1 protein. The antibodies which can be used in this type of assays can be polyclonal antibodies or sera, monoclonal antibodies, antibody fragments, such as Fv, Fab, Fab', F(ab')2, scFv (single chain Fv), diabodies, triabodies, tetrabodies, combibodies, etc., capable of recognizing and binding to proCOL11A1, or to an antigenic fragment thereof; said antibodies can be human antibodies, humanized antibodies or antibodies of non-human origin. Advantageously, the antibody recognizing the proCOL11A1 protein is an antibody specifically recognizing the VAR subdomain in the N-terminal propeptide of proCOL11A1. Illustrative examples of antibodies specifically detecting proCOL11A1 are mentioned in International Patent Application WO 2013/021088 A2. In a particular embodiment, said antibody is the monoclonal antibody 1E8.33 (García-Ocaña, et al., Int J Oncol. 2012; 40(5):1447-54), an antibody specifically recognizing the proCOL11A1 protein, without detecting other proteins with high sequence homology such as the COL5A1 protein, for example.

The antibody recognizing the proCOL11A1 protein can be, optionally, conjugated to a carrier. Likewise, said antibody recognizing the proCOL11A1 protein may be labeled or non-labeled; non-limiting illustrative examples of markers which can be used include radioctive isotopes, enzymes, fluorophores, chemiluminiscent reagents, enzyme cofactors or substrates, enzyme inhibitors, particles, dyes, etc. The non-labeled antibodies can be used in agglutination assays, whereas labeled antibodies can be used in various assays.

There is a large variety of assays well known by the persons skilled in the art which can be used for putting this particular embodiment of the first method of the invention [Method (A)] into practice, using non-labeled antibodies (primary antibody) recognizing the proCOL11A1 protein and labeled antibodies (secondary antibodies); these techniques include Western-blot, ELISA (enzyme-linked immunosorbent assay), RIA (radioimmunoassay), competitive EIA (enzyme immunoassay), DAS-ELISA (Double-antibody sandwich ELISA), immunohistochemical assays, multiplex detection techniques based on the use of protein microarrays, microspheres or biochips including specific antibodies, or assays based on colloidal precipitation, etc.

In a particular preferred embodiment, the detection of the presence of proCOL11A1 according to Method (A) is carried out by means of an immunohistochemical assay, a known histopathology method based on the use of a specific antibody, usually labeled with a marker (for example, an enzyme), which can transform a substrate into a visible compound without affecting the capacity of the antibody to form a complex with the antigen (proCOL11A1, in this case), applied to an organic tissue sample. With the use of some of the specific techniques (peroxidase, antiperoxidase, fluorescein, etc.), the antigen-antibody complex formed can be located and identified in the tissue or cell samples to be studied, whereby the antigenic markers characteristics of different cells are identified and the type of cell involved can be determined in the sample.

Briefly, in a particular embodiment of Method (A), the sample comprising ovary tissue to be analyzed for the purpose of detecting the presence of proCOL11A1 protein by means of an immunohistochemical assay, which can be a fresh sample, a frozen sample or a sample embedded in paraffin and fixed using a protecting agent (e.g., formalin or the like), is stained with a proCOL11A1-specific antibody (e.g., the monoclonal antibody 1E8.33 or a variant thereof) and the frequency of cells which have been stained and the intensity of the staining are determined. If necessary, the sample to be analyzed is subjected to a conditioning treatment which can include one or more of the following processes: fixing the tissue, obtaining sections having a suitable thickness, recovering the antigen, blocking molecules that may interfere in the indirect reaction, preventing non-specific bindings, etc. Advantageously, the detection of proCOL11A1 by means of said immunohistochemical assay in the sample to be analyzed is carried out in parallel with tissue or cell samples serving as positive marker and as negative marker, and, if desired, as reference, healthy tissues of the same origin as that the of the ovarian tumor or carcinoma being analyzed can be used. It is also common to use a background control. Typically, a value indicative of the total expression which is calculated depending on the frequency of stained cells and on the intensity in each of the stained cells is assigned to the sample. The typical criteria for assigning expression values to the samples were described in the Handbook of Immunohistochemistry and In situ Hybridization in Human Carcinomas, M. Hayat Ed., 2004, Academic Press, for example.

Example 1 describes an immunohistochemical assay for detecting the presence of proCOL11A1 in which the previously conditioned sample comprising ovary tissue to be analyzed is contacted with the monoclonal antibody targeting proCOL11A1 and, after the incubation period, it is developed with diaminobenzidine (DAB) using a suitable detection system.

The detection of the presence of proCOL11A1 by means of an immunohistochemical assay has several advantages since it is widely used in pathological anatomy laboratories, which enables the immediate applicability both of the first method of the invention and of the rest of the methods provided by the present invention by means of said technique, and, furthermore, it is highly automated, which makes it easier to perform same under homogeneous and reproducible conditions.

### Method (B)

In another particular embodiment, the first method of the invention comprises comparing the expression level of the proCOL11A1 protein in the sample from a subject under study with the expression level of said proCOL11A1 protein in a control sample, wherein an expression level of the proCOL11A1 protein in said sample greater than the expression level of said proCOL11A1 protein in a control sample is indicative that the ovarian tumor is a malignant tumor, or, alternatively an expression level of the proCOL11A1 protein in said sample equal to or less than the expression level of said proCOL11A1 protein in a control sample is indicative that the ovarian tumor is benign or in situ, or that the subject has a benign non-tumoral ovarian lesion.

The characteristics of the sample to be analyzed have already been mentioned above.

Method (B) comprises determining the expression level of the proCOL11A1 protein in a sample from a subject under study and comparing it with the expression level of said proCOL11A1 protein in a control sample.

The determination of the expression level of the proCOL11A1 protein is carried out by means of the quantification or determination of the amount or concentration of said proCOL11A1 protein in said sample.

The amount or concentration of the proCOL11A1 protein present in said samples can be quantified by means of any conventional method which allows detecting and quantifying said protein in the samples to be analyzed. In this case, Method (B) comprises performing an extraction step to obtain a protein extract containing said protein. The protein extracts can be obtained by means of conventional methods (Chomczynski et al., Anal. Biochem., 1987, 162:156; Chomczynski P., Biotechniques, 1993, 15:532).

Virtually any duly adapted conventional method for determining the amount or concentration of proteins in a sample can be used within the scope of the present invention for quantifying the amount or concentration of proCOL11A1 protein present in a sample. By way of non-limiting illustration, the amount or concentration of said proCOL11A1 protein can be determined by means of conventional methods, for example, by means of using antibodies capable of binding with proCOL11A1 and subsequently quantifying the complexes formed. In a particular embodiment, Method (B) comprises contacting the protein extract of the sample with a composition comprising one or more specific antibodies targeting one or more epitopes of the proCOL11A1 protein, under conditions allowing the formation of antibody:proCOL11A1 complexes and determining the amount or concentration of proCOL11A1 protein present in the sample. Non-limiting illustrative examples of said methods include methods based on the use of antibodies, affinity chromatography techniques, ligand binding assays, etc.

In a particular embodiment, the detection of the presence of proCOL11A1 is performed by means of an immunoassay based on the formation of an antigen-antibody type complex by means of using one (or more) antibodies recognizing one (or more) epitopes of proCOL11A1, and then viewing and quantifying the complexes formed by any suitable technique, including both radioctive and non-radioctive techniques such as, for example, colorimetric techniques, fluorometric techniques, luminiscent (e.g., bioluminiscent, chemiluminiscent, etc.) techniques, etc., using in turn secondary antibodies labeled with the suitable markers to that end.

The antibodies to be used for putting this particular embodiment of the first method of the invention [Method (B)] into practice are specific antibodies recognizing the proCOL11A1 protein, or an antigenic fragment of proCOL11A1, i.e., antibodies recognizing an epitope of proCOL11A1. The antibodies which can be used in this type of assays can be polyclonal sera, monoclonal antibodies, antibody fragments, such as Fv, Fab, Fab', F(ab')2, scFv (single chain Fv), diabodies, triabodies, tetrabodies, combibodies, etc., capable of recognizing and binding to proCOL11A1, or to an antigenic fragment thereof; said antibodies can be human antibodies, humanized antibodies or antibodies of non-human origin. Advantageously, the antibody recognizing the proCOL11A1 protein is an antibody specifically recognizing the VAR subdomain in the N-terminal propeptide of proCOL11A1. Illustrative examples of antibodies specifically detecting proCOL11A1 have been mentioned in relation to Method (A), the content of which is incorporated herein by reference. International Patent Application WO 2013/021088 A2 describes antibodies specifically recognizing the VAR subdomain in the N-terminal propeptide of proCOL11A1. In a particular embodiment, said antibody is the monoclonal antibody, clone 1E8.33, (García-Ocaña, et al., Int J Oncol. 2012; 40(5):1447-54).

The antibody recognizing proCOL11A1 can be, optionally, conjugated to a carrier. Likewise, said antibody recognizing proCOL11A1 can be labeled or non-labeled; non-limiting illustrative examples of markers which can be used include radioctive isotopes, enzymes, fluorophores, chemiluminiscent reagents, enzyme cofactors or substrates, enzyme inhibitors, particles, dyes, etc. The non-labeled antibodies can be used in agglutination assays, whereas labeled antibodies can be used in various assays.

There is a large variety of assays well known by the persons skilled in the art which can be used for putting this particular embodiment [Method (B)] of the first method of the invention into practice, using non-labeled antibodies (primary antibody) recognizing proCOL11A1 and labeled antibodies (secondary antibodies); these techniques include Western-blot, ELISA, RIA, competitive EIA, DAS-ELISA, immunohistochemical assays, multiplex detection techniques based on the use of protein microarrays, microspheres or biochips including specific antibodies, or assays based on colloidal precipitation, etc.

In a particular preferred embodiment, the detection and quantification of the proCOL11A1 protein according to Method (B) is carried out by means of an enzyme-linked immunosorbent assay (ELISA), an known immunodetection method based on the use of one or two specific antibodies, one of them usually labeled with an enzyme, which can transform a substrate into a visible compound without affecting the capacity of the antibody to form a complex with the antigen (proCOL11A1, in this case), applied to a protein extract generally originating from a biological fluid (e.g., blood, serum, urine, ascites fluid, etc.) or from a tissue portion, or applied to the biological fluid itself. With the use of some of the specific techniques (peroxidase, antiperoxidase, fluorescein, etc.), the antigen-antibody complex formed can be detected and quantified in the samples to be studied, the levels of protein to be analyzed in samples of different origin being able to be easily quantified. In a specific embodiment, monoclonal antibody 1E8.33 or a variant thereof such as the antibody, or one of the antibodies, recognizing the proCOL11A1 protein is used.

Briefly, in a particular embodiment of Method (B), the sample comprising a biological fluid for the purpose of detecting the presence of proCOL11A1 by means of an immunohistochemical assay, which can be a fresh sample or a frozen sample comprising blood, serum, urine, ascites fluid, etc., is contacted with labeled anti-proCOL11A1 antibody/antibodies (e.g., monoclonal antibody 1E8.33 or a variant thereof) and the concentration of the protein is quantified depending on the intensity of the enzymatic reaction associated with the antibody label. If necessary, the sample to be analyzed is subjected to a protein extraction process by means of conventional methods to concentrate the proteins, making detection thereof easier (Chomczynski et al., Anal. Biochem., 1987, 162:156; Chomczynski P., Biotechniques, 1993, 15:532). Advantageously, detection by means of said immunohistochemical assay of proCOL11A1 in the sample to be analyzed is carried out in parallel with samples containing known levels of proCOL11A1 as reference (standard curve), formed by human samples of known concentration, proCOL11A1 recombinant protein or extracts from cell lines expressing the protein, which will allow calculating the concentration of proCOL11A in the samples to be analyzed by comparing the intensities obtained.

The quantification of the proCOL11A1 protein by means of an ELISA-type immunodetection assay has several advantages since it is widely used in biochemical laboratories, which enables the immediate applicability not only of the first method of the invention, particularly Method (B), but also any of the other methods provided by this invention, by means of said technique, and, furthermore, it is highly automated, which makes it easier to perform same under the same conditions.

Method (B) comprises the step of comparing the amount or concentration of proCOL11A1 protein determined in the sample from the subject under study with the amount or concentration of proCOL11 protein of the control sample (reference value). In a particular embodiment, the control sample is a sample from subjects without clinical history of ovarian tumors.

Therefore, once the reference value has been established, the expression level of the proCOL11A1 protein in the sample from the subject under study is compared with the reference value. As a result of this comparison, the expression level of the proCOL11A1 protein in the sample from the subject can be "greater than" (higher than), "less than" ("lower than") or "equal to" said reference value for said protein in the control sample. As described above, in the context of the present invention, the expression level of the protein of interest (proCOL11A1) in the sample from the subject under study is considered "greater" than the reference value for said protein when the expression level of said protein in the sample from the subject under study increases, for example, by 3%, 5%, 10%, 25%, 50%, 100% or even more when compared with the reference value for said protein, or when it increases, for example, at least by 1.1-fold, 1.5-fold, 2-fold, 5-fold, 10-fold, 20-fold, 30-fold, 40-fold, 50-fold, 60-fold, 70-fold, 80-fold, 90-fold, 100-fold or even more when compared with the reference value for said protein. Likewise, in the context of the present invention, the expression level of the gene of interest in the sample from the subject is considered "less" than the reference value for said protein when the expression level of said protein in the sample from the subject under study decreases, for example, by 3%, 5%, 10%, 25%, 50%, 75%, or even 100% when compared with the reference value for said protein. Additionally, in the context of the present invention, the expression level of the gene of interest in the sample from the subject is considered "equal" to the reference value for said protein when the expression level of said protein in the sample from the subject under study is substantially the same as the reference value for said protein (i.e., the reference value + 3%).

2. Based on the detection of the presence of proCOL11A1 protein or of its expression level in a sample from a subject under study, it is possible to link the detection of the presence (or the non-detection of the presence-absence) of said proCOL11A1 protein in said sample, or the expression level of said proCOL-11A1 protein in said sample from said subject, with possible ovarian tumor malignancy or with the existence of a benign non-tumoral ovarian lesion.

Therefore, in a subsequent step, the first method of the invention involves correlating either the detection of the presence (or the non-detection of the presence-absence) of the proCOL11A1 protein in the sample from the subject under study with possible ovarian tumor malignancy or with the existence of a benign non-tumoral ovarian lesion [Method (A)] or correlating the expression level of the proCOL11A1 protein in the sample from the subject under study in comparison with the expression level of the proCOL11A1 protein in a control sample with possible ovarian tumor malignancy or with the existence of a benign non-tumoral ovarian lesion [Method (B)].

This correlation allows establishing that the ovarian tumor is a malignant ovarian tumor when:
- the presence of proCOL11A1 protein is detected in the sample from the subject under study, or when
- the expression level of the proCOL11A1 protein in the sample from the subject under study is greater than the expression level of said proCOL11A1 protein in a control sample.

Said correlation also allows establishing that the ovarian tumor is a benign ovarian tumor or an *in situ* ovarian tumor when:
- the presence of proCOL11A1 protein is not detected in the sample from the subject under study, or when
- the expression level of the proCOL11A1 protein in the sample from the subject under study is equal to or less than the expression level of said proCOL11A1 protein in a control sample.

Likewise, said correlation also allows establishing that the subject has a benign non-tumoral ovarian lesion when:
- the presence of proCOL11A1 protein is not detected in the sample from the subject under study, or when
- the expression level of the proCOL11A1 protein in the sample from the subject under study is equal to or less than the expression level of said proCOL11A1 protein in a control sample.

Therefore, in a particular embodiment, the first method of the invention allows differentiating between a malignant ovarian tumor and a benign or *in situ* ovarian tumor; i.e., it allows distinguishing a benign or *in situ* ovarian tumor from a malignant ovarian tumor. In another particular embodiment, the second method of the invention allows differentiating between a malignant ovarian tumor and a benign non-tumoral ovarian lesion; i.e., it allows distinguishing a benign non-tumoral ovarian lesion from a malignant ovarian tumor, or in other words, it allows excluding a benign non-tumoral ovarian lesion from the ovarian tumor or carcinoma diagnosis.

The meaning of the expression "detection of the presence of proCOL11A1 protein" or the like, as well as the meaning of an expression level of the proCOL11A1 protein "greater" than, "equal" to or "less" than that of the proCOL11A1 protein in a control sample, as it is used herein, has already been indicated above and is incorporated herein by reference. Likewise, in the sense used in this description, the expression "non-detection of the presence of proCOL11A1 protein" or the like refers to the incapability to detect the proCOL11A1 protein in the analyzed sample using any of the methods referenced to above.

The information provided by the first method of the invention can be used effectively by the specialist for selecting the most suitable treatment to be administered to the subject under study depending on the nature of the tumoral (benign/malignant) or non-tumoral lesion of the subject under study.

### 2. Method for the diagnosis or prognosis of ovarian tumor malignancy

In another aspect, the invention relates to an *in vitro* method for the diagnosis or prognosis of ovarian tumor malignancy in a subject, wherein said subject is a subject suspected of having a malignant ovarian tumor, wherein said malignant ovarian tumor is a malignant tumor in ovaries which can be serous, mucinous, endometrioid, clear cell tumor, or undifferentiated tumor, or is a subject diagnosed with a low malignant potential or borderline ovarian tumor, with or without extraovarian implantations hereinafter "second method of the invention", selected from Method (A) and Method (B), wherein
A) Method (A) comprises
   - detecting the presence of proCOL11A1 protein in a sample obtained by means of biopsy, cytology or surgical resection, from said subject suspected of having a malignant ovarian tumor or diagnosed with a low malignant potential or borderline ovarian tumor, with or without extraovarian implantations;
   wherein
   - the detection of the presence of proCOL11A1 protein in said sample from said subject suspected of having a malignant ovarian tumor or diagnosed with a low malignant potential or borderline ovarian tumor, with or without extraovarian implantations, is indicative that said subject has a malignant ovarian tumor or that said low malignant potential or borderline tumor, with or without extraovarian implantations, is capable of progressing into a malignant ovarian tumor, or, alternatively
   - the non-detection of the presence of proCOL11A1 protein in said sample from said subject suspected of having a malignant ovarian tumor or diagnosed with a low malignant potential or borderline ovarian tumor, with or without extraovarian implantations, is indicative that said subject does not have a malignant ovarian tumor, or that said subject has a benign ovarian tumor, or that said subject has an *in situ* ovarian tumor, or that said low malignant potential or borderline tumor is not capable of progressing into a malignant ovarian tumor; and
B) Method (B) comprises
   - comparing the expression level of the proCOL11A1 protein in said sample from said subject suspected of having a malignant ovarian tumor or diagnosed with a low malignant potential or borderline ovarian tumor, with or without extraovarian implantations, with the expression level of said proCOL11A1 protein in a control sample;
   wherein
   - an expression level of the proCOL11A1 protein in said sample from said subject suspected of having a malignant ovarian tumor or diagnosed with a low malignant potential or borderline ovarian tumor, with or without extraovarian implantations, greater than the expression level of said proCOL11A1 protein in a control sample is indicative that said subject has a malignant ovarian tumor or that said low malignant potential or borderline tumor is capable of progressing into a malignant ovarian tumor, or, alternatively
   - an expression level of the proCOL11A1 protein in said sample from said subject suspected of having a malignant ovarian tumor or diagnosed with a low malignant potential or borderline ovarian tumor, with or without extraovarian implantations, equal to or less than the expression level of said proCOL11A1 protein in a control sample is indicative that the subject does not have a malignant ovarian tumor, or that said subject has a benign ovarian tumor, or that said subject has an *in situ* ovarian tumor, or that said low malignant potential or borderline tumor is not capable of progressing into a malignant ovarian tumor.

The second method of the invention is a method of high sensitivity and specificity and is based on the fact that a subject with a malignant ovarian tumor or with a low malignant potential or borderline ovarian tumor, with or without extraovarian implantations, being capable of progressing into a malignant tumor, expresses and shows the proCOL11A1 protein or has high levels of proCOL11A1 protein in absolute terms or in comparison with the corresponding levels in control samples from subjects without clinical history of ovarian tumors, or with benign ovarian lesions or benign ovarian tumors.

Therefore according to the third method of the invention, the presence of proCOL11A1 protein is detected and/or the expression level of said proCOL11A1 protein is determined in a sample from the subject under study, and the obtained result is correlated with ovarian tumor malignancy or with whether or not a low malignant potential or borderline ovarian tumor, with or without extraovarian implantations, is capable of progressing into a malignant ovarian tumor.

The characteristics of the sample and the proCOL11A1 protein as well as the methods for detecting and/or quantifying said protein have already been mentioned above in relation to the first and second methods of the invention and are incorporated herein by reference.

According to the second method of the invention, the subject under study is a subject with a malignant ovarian tumor, wherein said malignant ovarian tumor is a malignant tumor in ovaries which can be serous, mucinous, endometrioid, clear cell tumor, or undifferentiated tumor, or a subject with a low malignant potential or borderline ovarian tumor, with or without extraovarian implantations.

Based on the detection/non-detection of the presence of proCOL11A1 or of its expression level in a sample from a subject under study, i.e., a subject suspected of having a malignant ovarian tumor or diagnosed with a low malignant potential or borderline ovarian tumor, with or without extraovarian implantations, it is possible to link the detection of the presence (or the non-detection of the presence-absence) of the proCOL11A1 protein in said sample with ovarian tumor malignancy or with whether or not the ovarian tumor is capable of malignization (progression of the tumor into a malignant tumor).

Therefore, in a subsequent step, the second method of the invention involves correlating either the detection/non-detection (absence) of the presence of proCOL11A1 protein [Method (A)], or correlating its expression level [Method (B)] with a diagnosis of malignancy or of whether or not the ovarian tumor is capable of malignization.

This correlation can indicate that:
- the ovarian tumor is malignant or is capable of progressing into a malignant tumor when the presence of proCOL11A1 protein is detected in the sample from the subject suspected of having a malignant ovarian carcinoma or diagnosed with a low malignant potential or borderline ovarian tumor, with or without extraovarian implantations, or when the expression level of said proCOL11A1 protein in the sample from the subject suspected of having a malignant ovarian tumor or diagnosed with a low malignant potential or borderline ovarian tumor, with or without extraovarian implantations, is greater than the expression level of said proCOL11A1 protein in a control sample, or
- the ovarian tumor is a benign or *in situ* tumor, or there is no tumor whatsoever in the ovary, or the low malignant potential or borderline ovarian tumor is not capable of progressing into a malignant tumor, when the presence of proCOL11A1 protein is not detected in the sample from the subject suspected of having a malignant ovarian tumor or diagnosed with a low malignant potential or borderline ovarian tumor, with or without extraovarian implantations, under study, or when the expression level of said proCOL11A1 protein in the sample from the subject suspected of having a malignant ovarian tumor or diagnosed with a low malignant potential or borderline ovarian tumor, with or without extraovarian implantations, is equal to or less than the expression level of said proCOL11A1 protein in a control sample.

In other words, this correlation allows establishing that the ovarian tumor in a subject suspected of having a malignant ovarian tumor is a malignant ovarian tumor when:
- the presence of proCOL11A1 protein is detected in the sample from the subject suspected of having a malignant ovarian tumor, or when
- the expression level of the proCOL11A1 protein in the sample from the subject suspected of having a malignant ovarian tumor is greater than the expression level of said proCOL11A1 protein in a control sample.

Said correlation also allows establishing that the low malignant potential or borderline ovarian tumor is capable of progressing into a malignant tumor when:
- the presence of proCOL11A1 protein is detected in the sample from the subject diagnosed with a low malignant potential or borderline ovarian tumor, with or without extraovarian implantations, or when
- the expression level of the proCOL11A1 protein in the sample from the subject diagnosed with a low malignant potential or borderline ovarian tumor, with or without extraovarian implantations, is greater than the expression level of said proCOL11A1 protein in a control sample.

Likewise, said correlation also allows establishing that the ovarian tumor in a subject suspected of having a malignant ovarian tumor is not a malignant ovarian tumor when:
- the presence of proCOL11A1 protein is not detected in the sample from the subject suspected of having a malignant ovarian tumor, or when
- the expression level of the proCOL11A1 protein in the sample from the subject suspected of having a malignant ovarian tumor is equal to or less than the expression level of said proCOL11A1 protein in a control sample.

Furthermore, said correlation also allows establishing that the low malignant potential or borderline ovarian tumor is not capable of progressing into a malignant tumor when:
- the presence of proCOL11A1 protein is not detected in the sample from the subject suspected of having a malignant ovarian tumor, or when
- the expression level of the proCOL11A1 protein in the sample from the subject suspected of having a malignant ovarian tumor is equal to or less than the expression level of said proCOL11A1 protein in a control sample.

Finally, this correlation together with other diagnostic tests also allows establishing that the subject suspected of having a malignant ovarian tumor or diagnosed with a low malignant potential or borderline ovarian tumor, with or without extraovarian implantations, does not have a malignant ovarian tumor but rather a non-tumoral ovarian lesion, a benign ovarian tumor, an *in situ* tumor or a borderline tumor without malignization probability, when:
- the presence of proCOL11A1 protein is not detected in the sample from the subject suspected of having a malignant ovarian tumor or diagnosed with a low malignant potential or borderline ovarian tumor, with or without extraovarian implantations, or when
- the expression level of the proCOL11A1 protein in the sample from the subject suspected of having a malignant ovarian tumor or diagnosed with a low malignant potential or borderline ovarian tumor, with or without extraovarian implantations, is equal to or less than the expression level of said proCOL11A1 protein in a control sample.

Therefore, the second method of the invention allows establishing if a subject suspected of having a malignant ovarian tumor, or diagnosed with a low malignant potential or borderline ovarian tumor, with or without extraovarian implantations, has a malignant ovarian tumor, or a benign ovarian tumor, or an *in situ* ovarian tumor, or whether or not the low malignant potential or borderline ovarian tumor is capable of progressing into a malignant tumor, or if said subject does not have any tumoral ovarian lesion but rather a non-tumoral ovarian lesion .

This information can be used effectively by the specialist for selecting the most suitable treatment to be administered to the subject suspected of having a malignant ovarian tumor or diagnosed with a low malignant potential or borderline ovarian tumor, with or without extraovarian implantations, depending on whether or not the tumor is malignant, or whether or not it is capable of progressing into a malignant tumor, or if the subject has a non-tumoral ovarian lesion.

### 3. Method for determining the malignization probability of a low malignant potential or borderline ovarian tumor

In another aspect, the invention relates to an *in vitro* method for determining the malignization probability of a low malignant potential or borderline ovarian tumor, with or without extraovarian implantations, in a subject diagnosed with said tumor, hereinafter "third method of the invention", selected from Method (A) and Method (B), wherein
A) Method (A) comprises
   - detecting the presence of proCOL11A1 protein in a sample obtained by means of biopsy, cytology or surgical resection from said subject diagnosed with a low malignant potential or borderline ovarian tumor, with or without extraovarian implantations;
   wherein
   - the detection of the presence of proCOL11A1 protein in said sample from said subject diagnosed with a low malignant potential or borderline ovarian tumor, with or without extraovarian implantations, is indicative that the probability of the tumor progressing into a malignant ovarian tumor is high, or, alternatively
   - the non-detection of the presence of proCOL11A1 protein in said sample from said subject diagnosed with a low malignant potential or borderline ovarian tumor, with or without extraovarian implantations, is indicative that the probability of the tumor progressing into a malignant ovarian tumor is low; and
B) Method (B) comprises
   - comparing the expression level of the proCOL11A1 protein in said sample from said subject diagnosed with a low malignant potential or borderline ovarian tumor, with or without extraovarian implantations, with the expression level of said proCOL11A1 protein in a control sample;
   wherein
   - an expression level of the proCOL11A1 protein in said sample from said subject diagnosed with a low malignant potential or borderline ovarian tumor, with or without extraovarian implantations, greater than the expression level of said proCOL11A1 protein in a control sample is indicative that the probability of the tumor progressing into a malignant ovarian tumor is high; or, alternatively
   - an expression level of the proCOL11A1 protein in said sample from said subject diagnosed with a low malignant potential or borderline ovarian tumor, with or without extraovarian implantations, equal to or less than the expression level of said proCOL11A1 protein in a control sample is indicative that the probability of the tumor progressing into a malignant ovarian tumor is low.

Therefore according to the third method of the invention, the presence of proCOL11A1 protein is detected and/or the expression level of said proCOL11A1 protein is determined in a sample from the subject under study, such as a subject diagnosed with a low malignant potential or borderline ovarian tumor, with or without extraovarian implantations, and the obtained result is correlated with the malignization probability of the tumor, i.e., with the probability of said tumor progressing into a malignant ovarian tumor.

The characteristics of the sample and the proCOL11A1 protein as well as the methods for detecting and/or quantifying said protein have already been mentioned above in relation to the first method of the invention and are incorporated herein by reference.

In this case, the subject under study is a subject diagnosed with a low malignant potential or borderline ovarian tumor, with or without extraovarian implantations.

Based on the detection of the presence of proCOL11A1 protein or of its expression level in a sample from said subject, it is possible to link the detection of the presence (or the non-detection of the presence-absence) of said proCOL11A1 protein in said sample, or the expression level of said proCOL-11A1 protein in said sample from said subject, with the probability of a low malignant potential or borderline ovarian tumor, with or without extraovarian implantations, progressing into a malignant ovarian tumor.

Therefore, in a subsequent step, the third method of the invention involves correlating either the detection of the presence (or the non-detection of the presence-absence) of the proCOL11A1 protein in the sample from the subject under study, with the probability of a low malignant potential or borderline ovarian tumor, with or without extraovarian implantations, progressing into a malignant ovarian tumor [Method (A)] or correlating the expression level of the proCOL11A1 protein in the sample from the subject under study in comparison with the expression level of the proCOL11A1 protein in a control sample, with the probability of a low malignant potential or borderline ovarian tumor, with or without extraovarian implantations, progressing into a malignant ovarian tumor [Method (B)].

This correlation allows establishing that the probability of a low malignant potential or borderline ovarian tumor, with or without extraovarian implantations, progressing into a malignant ovarian tumor is high when:
- the presence of proCOL11A1 protein is detected in the sample from the subject diagnosed with a low malignant potential or borderline ovarian tumor, with or without extraovarian implantations, or when
- the expression level of the proCOL11A1 protein in the sample from the subject diagnosed with a low malignant potential or borderline ovarian tumor, with or without extraovarian implantations, is greater than the expression level of said proCOL11A1 protein in a control sample.

This correlation also allows establishing that the probability of a low malignant potential or borderline ovarian tumor, with or without extraovarian implantations, progressing into a malignant ovarian tumor is low when:
- the presence of proCOL11A1 protein is not detected in the sample from the subject diagnosed with a low malignant potential or borderline ovarian tumor, with or without extraovarian implantations, or when
- the expression level of the proCOL11A1 protein in the sample from the subject diagnosed with a low malignant potential or borderline ovarian tumor, with or without extraovarian implantations, is equal to or less than the expression level of said proCOL11A1 protein in a control sample.

Generally, when the malignization probability of a low malignant potential or borderline ovarian tumor, with or without extraovarian implantations, is high, said tumor will progress into a malignant ovarian tumor; likewise, generally, when the malignization probability of a low malignant potential or borderline ovarian tumor, with or without extraovarian implantations, is low, said tumor will not progress into a malignant ovarian tumor.

Therefore, in a particular embodiment, the third method of the invention allows establishing the probability of a low malignant potential or borderline ovarian tumor, with or without extraovarian implantations, progressing into a malignant ovarian tumor.

The meaning of the terms "probability", "malignization", as well as the meaning of the terms "high" and "low" applied to probability, as it is used herein, has already been indicated above and is incorporated herein by reference. The expressions "detection of the presence of proCOL11A1 protein", or the like, and "non-detection of the presence of proCOL11A1 protein" or the like, have also been defined above.

The information provided by the third method of the invention can be used effectively by the specialist for selecting the most suitable treatment to be administered to the subject under study depending on the probability of a low malignant potential or borderline ovarian tumor, with or without extraovarian implantations, progressing into a malignant ovarian tumor.

### 4. Methods for selecting treatments

There are different treatments which can be applied on the subject suspected of having a malignant ovarian tumor or diagnosed with a low malignant potential or borderline ovarian tumor, with or without extraovarian implantations, depending on whether or not said subject has a malignant tumor or on whether or not the tumor is capable of progressing into a malignant tumor. Generally, patients having malignant tumors are subjected to a radical surgery to eliminate the affected tissue entirely, and are also subjected to additional tests for assessing the tumor infiltration level and the presence of metastasis. The degree of tumor infiltration determines the chemotherapy or radiotherapy treatments necessary for each patient, as well as the follow-up to which the patient will be subjected. In the case of benign and *in situ* tumors, the tumor resection can be carried out by means of conservative surgery, and additional tests for detecting metastasis are not indicated. In the case of low malignant potential or borderline tumors, if the latter are not capable of progressing into malignant tumors, they may be treated as a benign tumor, but if they are capable of progressing, they must be treated as a malignant tumor at least as regards the surgery, completely eliminating the tumor and the extraovarian implants, if any.

It is sometimes complicated to differentiate benign and *in situ* tumors from malignant tumors with current tumor classification methods due to the morphological and structural similarities of the tumors and to the fact that different types of tumor often appear in the same sample. In the case of low malignant potential or borderline tumors, there is no tool available today that allows predicting the progress of said tumors into malignant carcinomas.

Now it has been observed that, because it is possible to correlate the detection of the presence of, and/or the expression level of, the proCOL11A1 protein in a sample from a subject suspected of having a malignant ovarian tumor with the diagnosis of tumor malignancy in the tissue extracted by means of biopsy, the specialist can correctly classify the malignant tumors. Furthermore, the detection of the proCOL11A1 protein has also been linked with whether or not low malignant potential or borderline tumors are capable of progressing into a malignant tumor, being useful for classifying said tumors according to the risk of progression thereof. Therapeutic care for the subject is thus optimized and sped up and drawbacks associated with the application of insufficient or excessive surgery or post-surgery treatment are prevented, and additional surgical interventions are prevented, with the subsequent economical repercussion that it entails.

Therefore in another aspect, the invention relates to a method for selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment, or for selecting a subject diagnosed with a low malignant potential or borderline ovarian tumor, with or without extraovarian implantations, for treatment, wherein said treatment comprises surgically removing said ovarian tumor, performing additional analyses to evaluate tumor infiltration and metastasis if the subject is suspected of having a malignant ovarian tumor, choosing a chemotherapy and/or radiotherapy treatment, and the subsequent follow-up, hereinafter "fourth method of the invention", said method comprising:
- detecting the presence of proCOL11A1 protein in a sample obtained by means of biopsy, cytology or surgical resection from said subject suspected of having a malignant ovarian tumor, or from said subject diagnosed with a low malignant potential or borderline ovarian tumor, with or without extraovarian implantations; or
- comparing the expression level of the proCOL11A1 protein in said sample from said subject suspected of having a malignant ovarian tumor, or in said sample from said subject diagnosed with a low malignant potential or borderline ovarian tumor, with or without extraovarian implantations, with the expression level of said proCOL11A1 protein in a control sample;
wherein said subject is selected for said treatment:
- if the presence of proCOL11A1 protein is detected in said sample from the subject suspected of having a malignant ovarian tumor, or in said sample from said subject diagnosed with a low malignant potential or borderline ovarian tumor, with or without extraovarian implantations; or
- if the expression level of the proCOL11A1 protein in said sample from said subject suspected of having a malignant ovarian tumor, or in said sample from said subject diagnosed with a low malignant potential or borderline ovarian tumor, with or without extraovarian implantations, is greater than the expression level of said proCOL11A1 protein in the control sample; or, alternatively,
wherein said subject is not selected for said treatment:
- if the presence of proCOL11A1 protein is not detected in said sample from said subject suspected of having a malignant ovarian tumor, or in said sample from said subject diagnosed with a low malignant potential or borderline ovarian tumor, with or without extraovarian implantations; or
- if the expression level of the proCOL11A1 protein in said sample from said subject suspected of having a malignant ovarian tumor, or in said sample from said subject diagnosed with a low malignant potential or borderline ovarian tumor, with or without extraovarian implantations, is equal to or less than the expression level of said proCOL11A1 protein in the control sample.

Therefore according to the fourth method of the invention, the presence/absence of the proCOL11A1 protein is detected or the expression level of said proCOL11A1 protein is determined in a sample from the subject suspected of having a malignant ovarian tumor, or in a sample from a subject diagnosed with a low malignant potential or borderline ovarian tumor, with or without extraovarian implantations, which is being evaluated and the obtained result is correlated with the possibility of selecting said subject to receive a treatment which comprises completely removing the tumor, performing additional analyses to evaluate the degree of tumor invasion, i.e., of tumor infiltration and possible metastasis that may exist; choosing the chemotherapy and/or radiotherapy treatment to be applied to the subject; and the subsequent follow-up of the subject.

The characteristics of the sample and the proCOL11A1 protein as well as the methods for detecting and/or quantifying said protein have already been mentioned above in relation to the first method of the invention and are incorporated herein by reference. In this case, the subject under study is a subject suspected of having a malignant ovarian tumor or diagnosed with a low malignant potential or borderline ovarian tumor, with or without extraovarian implantations.

Once the presence/absence or the amount of proCOL11A1 protein in the sample from the subject suspected of having a malignant ovarian tumor, or diagnosed with a low malignant potential or borderline ovarian tumor, with or without extraovarian implantations, under study is analyzed, it is possible to evaluate the possibility of selecting said subject to receive a treatment, hereinafter "Treatment", which comprises completely removing the tumor, performing additional analyses to evaluate tumor infiltration and metastasis if the subject is suspected of having a malignant ovarian tumor, choosing a chemotherapy and/or radiotherapy treatment and the subsequent follow-up of the patient (subject)

The ovarian tumor can be completely removed by means of any method which allows assuring the complete removal of the tumor and/or of the affected area; non-limiting illustrative examples of such methods include cytoreduction, hysterectomy, adnexectomy, colostomy, lymph node removal, etc.

The analyses to be performed for the purpose of evaluating the degree of ovarian tumor invasion and determining tumor infiltration and metastasis include, among others, lymph nodes sampling to search for tumor presence, and image analysis by suitable techniques such as, for example, ultrasound, CAT scan, etc., to search for the points of metastasis.

In view of the obtained results, the specialist may choose a chemotherapy and/or radiotherapy treatment. In a particular embodiment, said treatment comprises a chemotherapy treatment. In a specific embodiment, said chemotherapy treatment comprises the administration of a suitable anti-tumor drug; non-limiting illustrative examples of such anti-tumor drugs include paclitaxel, altretamine, capecitabine, cyclophosphamide, etoposide, gemcitabine, doxorubicin, irinotecan, topotecan, etc. In another particular embodiment, said treatment comprises a radiotherapy treatment. In another particular embodiment, said treatment comprises a chemotherapy treatment followed by a subsequent radiotherapy treatment.

The treatment further comprises the subsequent follow-up of the patient for which the necessary diagnostic tests will be performed periodically to search for tumor relapses or the onset of metastasis, such as using imaging techniques or analyzing serum markers, such as tumor marker CA-125 (also referred to as "mucin 16" or "MUC16"), for example.

The possibility of selecting a subject to receive said Treatment is established based on the detection of the presence/absence of the proCOL11A1 protein or on the expression level of said protein in a sample from the subject suspected of having a malignant ovarian tumor or diagnosed with a low malignant potential or borderline ovarian tumor, with or without extraovarian implantations, under study, such that if the presence of proCOL11A1 protein is detected in said sample from the subject suspected of having a malignant ovarian tumor or diagnosed with a low malignant potential or borderline ovarian tumor, with or without extraovarian implantations, or if the expression level of the proCOL11A1 protein in said sample from the subject suspected of having a malignant ovarian tumor or diagnosed with a low malignant potential or borderline ovarian tumor, with or without extraovarian implantations, is greater than the expression level of said proCOL11A1 protein in the control sample, then said subject is selected for said Treatment. In contrast, i.e., if the presence of proCOL11A1 protein is not detected in the sample from the subject under study (subject suspected of having a malignant ovarian tumor or diagnosed with a low malignant potential or borderline ovarian tumor with or without extraovarian implantations) or if the expression level of the proCOL11A1 protein in said sample from the subject under study is equal to or less than the expression level of said proCOL11A1 protein in the control sample, then said subject is not selected for said Treatment.

The fourth method of the invention therefore allows selecting a subject suspected of having a malignant ovarian tumor or diagnosed with a low malignant potential of borderline ovarian tumor, with or without extraovarian implantations to receive said Treatment, a treatment which comprises completely removing the tumor, performing additional analyses to evaluate tumor infiltration and metastasis, applying the chosen chemotherapy and/or radiotherapy treatment and the subsequent follow-up of the subject. Therefore, if the presence of proCOL11A1 protein is detected in the analyzed sample from the subject, or if the expression level of the proCOL11A1 protein in said sample is greater than the expression level of said proCOL11A1 protein in a control sample, then said subject is a suitable candidate *a priori* for receiving said Treatment, whereas if the presence of proCOL11A1 protein is not detected in the analyzed sample from the subject, or if the expression level of the proCOL11A1 protein in said sample is equal to or less than the expression level of said proCOL11A1 protein in a control sample, then said subject is not a suitable candidate *a priori* for receiving Treatment.

This fourth method of the invention is a valuable tool for the treatment of ovarian tumors since, in view of the results provided by evaluating ovarian tumor malignancy by means of a method such as any of the first to third methods provided by this invention, the specialist can select the subject who is being evaluated to receive said Treatment which comprises completely removing the tumor, performing additional analyses to evaluate tumor infiltration and metastasis, applying the chosen chemotherapy and/or radiotherapy treatment and the subsequent follow-up, depending on the detection of the presence/absence of the proCOL11A1 protein, or on the expression level of the proCOL11A1 protein in the sample from said subject. Therefore, the methods and means provided by the present invention can aid the specialists in selecting the most suitable treatment to be administered to a subject having a malignant ovarian tumor and preventing adverse effects associated with delay in applying the suitable treatment as a result of an incorrect first diagnosis, or associated with the application of an overly aggressive treatment in subjects with benign or *in situ* tumors, or in low malignant potential or borderline tumors that are not capable of progressing into malignant tumors.

The fact that the subject under study is not selected to receive Treatment can mean that the ovarian lesion is not a malignant tumor but rather, for example, a non-tumoral benign lesion, a benign tumor, an *in situ* tumor or a borderline tumor, with or without extraovarian implantations, without probability of progressing into a malignant tumor, which can be treated by means of a treatment different from Treatment, which may or may not consist of the surgical resection of the lesion, an optional pharmacological treatment and the absence of a subsequent follow-up for detecting relapses or progression of the lesion.

The fact that the subject under study is not selected to receive Treatment can mean that the borderline tumor has no probability of progressing into a malignant tumor, and can be treated by means of a treatment different from Treatment 2, which may or may not consist of the surgical resection of the tumor, an optional pharmacological treatment and the absence of a subsequent follow-up for detecting relapses or progression of the lesion.

### 6. Method for selecting follow-ups

There are different follow-ups which can be applied on the subject suspected of having a malignant ovarian tumor or diagnosed with a low malignant potential or borderline ovarian tumor, with or without extraovarian implantations, depending on whether or not said subject has a malignant tumor or on whether or not the tumor is capable of progressing into a malignant tumor. As mentioned above, the patients having malignant tumors are usually subjected to a radical surgery to eliminate the affected tissue entirely, and also to additional tests for assessing the tumor infiltration level and the presence of metastasis, the result of which determines the chemotherapy and/or radiotherapy treatment to be administered to the patient, as well as the follow-up up to which the patient will be subjected. In the case of benign or *in situ* tumors, the tumor resection can be carried out by means of conservative surgery and additional tests for detecting metastasis are generally not indicated, although performing a follow-up of the disease is advisable. In the case of low malignant potential or borderline tumors, if the latter are not capable of progressing into malignant tumors, they may be treated as benign tumors, but if they are capable of progressing into malignant tumors, they must be treated as malignant tumors at least as regards the surgery, completely eliminating the tumor and the extraovarian implants, if any; in any case, performing a follow-up of the subject is advisable to prevent or treat possible relapses as soon as possible.

Now it has been observed that it is possible to correlate the detection of the presence of, and/or the expression level of, the proCOL11A1 protein in a sample from a subject suspected of having a malignant ovarian tumor with the diagnosis of tumor malignancy in the tissue extracted by means of biopsy, as well as with whether or not a low malignant potential or borderline tumor is capable of progressing into a malignant tumor, so the specialist can correctly classify the tumoral or non-tumoral ovarian lesions, establish the treatment to be applied and design the corresponding follow-up.

Therefore, in another aspect, the invention relates to a method for selecting a subject diagnosed with a low malignant potential or borderline ovarian tumor, with or without extraovarian implantations, for follow-up, wherein said follow-up comprises surgically resectioning said tumor and monitoring tumor recurrence, hereinafter "fifth method of the invention", said method comprising:
- detecting the presence of proCOL11A1 protein in a sample obtained by means of biopsy, cytology or surgical resection from said subject diagnosed with a low malignant potential or borderline ovarian tumor, with or without extraovarian implantations; or
- comparing the expression level of the proCOL11A1 protein in said sample from said subject diagnosed with a low malignant potential or borderline ovarian tumor, with or without extraovarian implantations, with the expression level of said proCOL11A1 protein in a control sample;
wherein said subject is selected for said follow-up:
- if the presence of proCOL11A1 protein is detected in said sample from the subject diagnosed with a low malignant potential or borderline ovarian tumor, with or without extraovarian implantations; or
- if the expression level of the proCOL11A1 protein in said sample from said subject diagnosed with a low malignant potential or borderline ovarian tumor; with or without extraovarian implantations, is greater than the expression level of said proCOL11A1 protein in the control sample.

Therefore according to the fifth method of the invention, the presence/absence of the proCOL11A1 protein is detected or the expression level of said proCOL11A1 protein is determined in a sample from the subject diagnosed with a low malignant potential or borderline ovarian tumor, with or without extraovarian implantations, which is being evaluated and the obtained result is correlated with the possibility of selecting said subject to be subjected to a follow-up which comprises surgically resectioning said tumor and monitoring tumor recurrence.

The characteristics of the sample and the proCOL11A1 protein as well as the methods for detecting and/or quantifying said protein have already been mentioned above in relation to the first method of the invention and are incorporated herein by reference. In this case, the subject under study is a subject diagnosed with a low malignant potential or borderline ovarian tumor, with or without extraovarian implantations.

Once the presence/absence or the amount of proCOL11A1 protein in the sample from the subject diagnosed with a low malignant potential or borderline ovarian tumor, with or without extraovarian implantations, under study is analyzed, it is possible to evaluate the possibility of selecting said subject to be subjected to a follow-up, hereinafter "Follow-up", which comprises surgically resectioning the tumor and monitoring tumor recurrence.

In the event that the presence of proCOL11A1 protein is detected in a sample from the subject under study, or in the event that the expression level of the proCOL11A1 protein in a sample from said subject is greater than the expression level of proCOL11A1 in a control sample, then the subject diagnosed with a low malignant potential or borderline ovarian tumor, with or without extraovarian implantations, can be selected to be subjected to Follow-up. In this case, since the tumor would have a high probability of progressing into a malignant ovarian tumor, then the follow-up comprises surgically resectioning the tumor by means of any suitable surgical method which allows completely eliminating or removing the tumor (for example, by means of any of the methods indicated in relation to the fifth method of the invention), and monitoring tumor recurrence, for which the necessary periodic tests would be performed either by means of image analysis by suitable techniques such as ultrasound, CAT scan, etc., or by means of analyzing serum markers, such as, tumor marker CA-125 ("MUC16"), for example, which allows detecting tumor relapses.

In view of the results obtained by means of Follow-up, the specialist can therefore in turn select the suitable treatment.

The possibility of selecting a subject to be subjected to said Follow-up is established based on the detection of the presence/absence of the proCOL11A1 protein or on the expression level of said protein in a sample from the subject diagnosed with a low malignant potential or borderline ovarian tumor, with or without extraovarian implantations, such that if the presence of proCOL11A1 protein is detected in said sample from the subject, or if the expression level of the proCOL11A1 protein in said sample from the subject is greater than the expression level of said proCOL11A1 protein in the control sample, then said subject is selected to be subjected to said Follow-up. In contrast, i.e., if the presence of proCOL11A1 protein is not detected in the sample from the subject under study or if the expression level of the proCOL11A1 protein in said sample from the subject under study is equal to or less than the expression level of said proCOL11A1 protein in the control sample, then said subject diagnosed with a low malignant potential or borderline ovarian tumor, with or without extraovarian implantations, is not selected to be subjected to said Follow-up.

The fifth method of the invention therefore allows selecting a subject diagnosed with a low malignant potential or borderline ovarian tumor, with or without extraovarian implantations, to be subjected to said Follow-up, a follow-up which comprises surgically resectioning the tumor and monitoring tumor recurrence. Therefore, if the presence of proCOL11A1 protein is detected in the analyzed sample from the subject, or if the expression level of the proCOL11A1 protein in said sample is greater than the expression level of said proCOL11A1 protein in a control sample, then said subject is a suitable candidate *a priori* to be subjected to said Follow-up.

This fifth method of the invention is also a valuable tool for the treatment of ovarian tumors since, in view of the results provided by evaluating ovarian tumor malignancy by means of a method such as any of the previous methods provided by this invention, the specialist can select the subject who is being evaluated to be subjected to said Follow-up depending on the detection of the presence/absence of the proCOL11A1 protein, or on the expression level of the proCOL11A1 protein in the sample from said subject. Therefore, the methods and means provided by the present invention can aid the specialists in selecting the most suitable follow-up to which a subject having a malignant ovarian tumor is subjected with the advantages that it entails.

### Uses

In another aspect, the invention relates to the use of the proCOL11A1 protein as a marker for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

In another aspect, the invention relates to the use of a specific antibody recognizing the proCOL11A1 protein for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or in situ ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

The characteristics of the specific antibody recognizing the proCOL11A1 protein, or a fragment thereof recognizing the proCOL11A1 protein, have been mentioned in relation to the first method of the invention, particularly in relation to Method (A), and are incorporated herein by reference. Advantageously, the antibody recognizing the proCOL11A1 protein is an antibody specifically recognizing the VAR subdomain in the N-terminal propeptide of proCOL11A1. Illustrative examples of antibodies specifically detecting proCOL11A1 are mentioned in International Patent Application WO 2013/021088 A2. In a particular embodiment, said antibody is the monoclonal antibody 1E8.33 (García-Ocaña, et al., Int J Oncol. 2012; 40(5):1447-54), an antibody specifically recognizing the proCOL11A1 protein, without detecting other proteins with high sequence homology such as the COL5A1 protein.

In another aspect, the invention relates to the use of a kit comprising a reagent recognizing the proCOL11A1 protein, or a reagent for the detection and/or quantification of the expression of the proCOL11A1 protein, for:
a) detecting a malignant ovarian tumor, wherein said malignant ovarian tumor is a malignant tumor in ovaries which can be serous, mucinous, endometrial, clear cell tumor, or undifferentiated tumor; or for
b) performing differential diagnosis of a malignant ovarian tumor, wherein said malignant ovarian tumor is a malignant tumor in ovaries which can be serous, mucinous, endometrial, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrial, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrial, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

In a particular embodiment, said reagent recognizing the proCOL11A1 protein is a specific antibody recognizing the proCOL11A1 protein, or a fragment thereof recognizing the proCOL11A1 protein. The characteristics of said specific antibody recognizing the proCOL11A1 protein, or a fragment thereof recognizing the proCOL11A1 protein, have been mentioned in relation to the first method of the invention, particularly in relation to Method (A) and are incorporated herein by reference. Advantageously, the antibody recognizing the proCOL11A1 protein is an antibody specifically recognizing the VAR subdomain in the N-terminal propeptide of proCOL11A1. Illustrative examples of antibodies specifically detecting proCOL11A1 are mentioned in International Patent Application WO 2013/021088 A2. In a particular embodiment, said antibody is the monoclonal antibody 1E8.33 (García-Ocaña, et al., Int J Oncol. 2012; 40(5):1447-54), an antibody specifically recognizing the proCOL11A1 protein, without detecting other proteins with high sequence homology such as the COL5A1 protein.

The following example illustrates the invention and must not be considered as limiting same.

### Example 1

### Differential expression of proCOL11A1 in ovarian tumors and the relation thereof with tumor malignancy

### 1.1 Materials and Methods

### Immunohistochemistry

Sections of the biopsies fixed in formalin and embedded in paraffin from patients suspected of having an ovarian tumor were made with microtome. The sections of 3-4 µm were dried throughout the night at 54-56°C in an oven. A pre-treatment with CC2 [Cell Conditioning 2] buffer [Ventana Medical Systems, Inc.; catalog no. 950-123] was applied at 98°C for 32 minutes. After titrating to adjust the suitable antibody concentration of 65, 26 and 13 µg/ml, the preparations were incubated with the proCOL11A1-specific monoclonal antibody 1E8.33 (WO 2013/021088) at a concentration of 26 µg/ml in an antibody diluent (Ventana-Roche, Tucson, Arizona) for 32 minutes at room temperature. The Optiview detection system (Ventana) was used and it was developed with diaminobenzidine (DAB) (Ventana).

Thirty-one cases of patients suspected of having ovarian tumor were analyzed with the proCOL11A1-specific monoclonal antibody 1E8.33. The immunostaining was evaluated with a double-blind assay by 2 different observers. It was considered positive when at least one cell with fibroblastic morphology had immunoprecipitate.

### 1.2 Analysis of the statistical data

Fisher's exact test was used to calculate frequency distribution. p<0.05 was considered significant.

### 1.3 Results

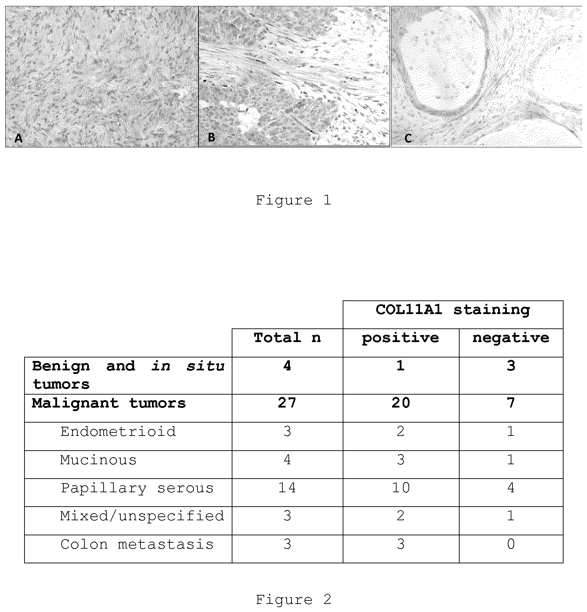

The expression of the proCOL11A1 protein in the histology sections of biopsies of the ovary from patients suspected of having an ovarian tumor was studied by means of using a proCOL11A1 protein-specific antibody (1E8.33). The objective of these experiments was to determine if the presence of proCOL11A1 immunolabeling could be associated with the presence of a malignant ovarian tumor. From the 31 analyzed cases, 21 were positive for immunolabeling with the proCOL11A1 protein-specific antibody, i.e., they showed the expression of the proCOL11A1 protein, and 10 were negative (Figure 1).

From the 21 cases which were positive for proCOL11A1 immunolabeling, 20 patients were diagnosed based on the biopsy with malignant ovarian tumor: 2 endometrioid adenocarcinomas, 3 mucinous adenocarcinomas, 2 mixed or unclassified carcinomas, 10 papillary serous carcinomas, and 3 colon metastasis to ovary. One of the samples which were positive for proCOL11A1 immunolabeling was clinically diagnosed in the biopsy with a benign tumor (Figure 2).

From the 10 negative cases without the expression of proCOL11A1, 7 cases corresponded to malignant ovarian tumor and 3 cases corresponded to benign or *in situ* ovarian tumors.

According to the Fischer's test, the frequency of positive staining for proCOL11A1 had statistically significant differences (p<0.05) between the group of patients with malignant ovarian tumors and the group of patients with benign and *in situ* ovarian tumors.

Furthermore, the sensitivity for detecting malignant ovarian tumors in biopsy tissues (74%), and the specificity for detecting tumor infiltration in biopsy cores (75%) as well as the positive predictive values (PPVs) and negative predictive values (NPVs) for diagnosing infiltration (PPV 95% and NPV 30%), were calculated. These results demonstrate that this marker (proCOL11A1) has a high sensitivity and specificity for detecting malignant ovarian tumors.

### 1.4 Conclusions

Immunostaining with the proCOL11A1-specific monoclonal antibody 1E8.33 shows high sensitivity and specificity in the detection of malignant ovarian tumors.

## Claims

1. An *in vitro* method for the differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion, in a subject, selected from Method (A) and Method (B), wherein
A) Method (A) comprises:
detecting the presence of proCOL11A1 protein in a sample obtained by means of biopsy, cytology or surgical resection from said subject wherein
- the detection of the presence of proCOL11A1 protein in said sample is indicative that the ovarian tumor is malignant; or, alternatively
- the non-detection of the presence of proCOL11A1 protein in said sample is indicative that the ovarian tumor is benign or *in situ,* or that the subject has a benign non-tumoral ovarian lesion; and
B) Method (B) comprises:
- comparing the expression level of the proCOL11A1 protein in said sample with the expression level of said proCOL11A1 protein in a control sample;
wherein
- an expression level of the proCOL11A1 protein in said sample greater than the expression level of said proCOL11A1 protein in a control sample is indicative that the ovarian tumor is a malignant tumor, or, alternatively
- an expression level of the proCOL11A1 protein in said sample equal to or less than the expression level of said proCOL11A1 protein in a control sample is indicative that the ovarian tumor is benign or *in situ,* or that the subject has a benign non-tumoral ovarian lesion.

2. An *in vitro* method for the diagnosis or prognosis of ovarian tumor malignancy in a subject, wherein said subject is a subject suspected of having a malignant ovarian tumor, wherein said malignant ovarian tumor is a malignant tumor in ovaries which can be serous, mucinous, endometrioid, clear cell tumor, or undifferentiated tumor, or is a subject diagnosed with a low malignant potential or borderline ovarian tumor, with or without extraovarian implantations, selected from Method (A) and Method (B), wherein
A) Method (A) comprises
- detecting the presence of proCOL11A1 protein in a sample obtained by means of biopsy, cytology or surgical resection , from said subject suspected of having a malignant ovarian tumor or diagnosed with a low malignant potential or borderline ovarian tumor, with or without extraovarian implantations;
wherein
- the detection of the presence of proCOL11A1 protein in said sample from said subject suspected of having a malignant ovarian tumor or diagnosed with a low malignant potential or borderline ovarian tumor, with or without extraovarian implantations, is indicative that said subject has a malignant ovarian tumor or that said low malignant potential or borderline tumor, with or without extraovarian implantations, is capable of progressing into a malignant ovarian tumor, or, alternatively
- the non-detection of the presence of proCOL11A1 protein in said sample from said subject suspected of having a malignant ovarian tumor or diagnosed with a low malignant potential or borderline ovarian tumor, with or without extraovarian implantations, is indicative that said subject does not have a malignant ovarian tumor, or that said subject has a benign or *in situ* ovarian tumor, or that said low malignant potential or borderline tumor is not capable of progressing into a malignant ovarian tumor; and
B) Method (B) comprises
- comparing the expression level of the proCOL11A1 protein in said sample from said subject suspected of having a malignant ovarian tumor or diagnosed with a low malignant potential or borderline ovarian tumor, with or without extraovarian implantations, with the expression level of said proCOL11A1 protein in a control sample;
wherein
- an expression level of the proCOL11A1 protein in said sample from said subject suspected of having a malignant ovarian tumor or diagnosed with a low malignant potential or borderline ovarian tumor, with or without extraovarian implantations, greater than the expression level of said proCOL11A1 protein in a control sample is indicative that said subject has a malignant ovarian tumor or that said low malignant potential or borderline tumor is capable of progressing into a malignant ovarian tumor, or, alternatively
- an expression level of the proCOL11A1 protein in said sample from said subject suspected of having a malignant ovarian tumor or diagnosed with a low malignant potential or borderline ovarian tumor, with or without extraovarian implantations, equal to or less than the expression level of said proCOL11A1 protein in a control sample is indicative that the subject does not have a malignant ovarian tumor, or that said subject has a benign or *in situ* ovarian tumor, or that said low malignant potential or borderline tumor is not capable of progressing into a malignant ovarian tumor.

3. An *in vitro* method for determining the malignization probability of a low malignant potential or borderline ovarian tumor, with or without extraovarian implantations, in a subject diagnosed with said tumor, selected from Method (A) and Method (B), wherein
A) Method (A) comprises
- detecting the presence of proCOL11A1 protein in a sample obtained by means of biopsy, cytology or surgical resection from said subject diagnosed with a low malignant potential or borderline ovarian tumor, with or without extraovarian implantations;
wherein
- the detection of the presence of proCOL11A1 protein in said sample from said subject diagnosed with a low malignant potential or borderline ovarian tumor, with or without extraovarian implantations, is indicative that the probability of the tumor progressing into a malignant ovarian tumor is high, or, alternatively
- the non-detection of the presence of proCOL11A1 protein in said sample from said subject diagnosed with a low malignant potential or borderline ovarian tumor, with or without extraovarian implantations, is indicative that the probability of the tumor progressing into a malignant ovarian tumor is low; and
B) Method (B) comprises
- comparing the expression level of the proCOL11A1 protein in said sample from said subject diagnosed with a low malignant potential or borderline ovarian tumor, with or without extraovarian implantations, with the expression level of said proCOL11A1 protein in a control sample;
wherein
- an expression level of the proCOL11A1 protein in said sample from said subject diagnosed with a low malignant potential or borderline ovarian tumor, with or without extraovarian implantations, greater than the expression level of said proCOL11A1 protein in a control sample is indicative that the probability of the tumor progressing into a malignant ovarian tumor is high; or, alternatively
- an expression level of the proCOL11A1 protein in said sample from said subject diagnosed with a low malignant potential or borderline ovarian tumor, with or without extraovarian implantations, equal to or less than the expression level of said proCOL11A1 protein in a control sample is indicative that the probability of the tumor progressing into a malignant ovarian tumor is low.

4. A method for selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment, or for selecting a subject diagnosed with a low malignant potential or borderline ovarian tumor, with or without extraovarian implantations, for treatment, wherein said treatment comprises surgically removing said ovarian tumor, performing additional analyses to evaluate tumor infiltration and metastasis if the subject is suspected of having a malignant ovarian tumor, choosing a chemotherapy and/or radiotherapy treatment, and the subsequent follow-up, said method comprising:
- detecting the presence of proCOL11A1 protein in a sample obtained by means of biopsy, cytology or surgical resection from said subject suspected of having the malignant ovarian tumor, or from said subject diagnosed with a low malignant potential or borderline ovarian tumor, with or without extraovarian implantations; or
- comparing the expression level of the proCOL11A1 protein in said sample from said subject suspected of having a malignant ovarian tumor, or in said sample from said subject diagnosed with a low malignant potential or borderline ovarian tumor, with or without extraovarian implantations, with the expression level of said proCOL11A1 protein in a control sample;
wherein said subject is selected for said treatment:
- if the presence of proCOL11A1 protein is detected in said sample from the subject suspected of having a malignant ovarian tumor, or in said sample from said subject diagnosed with a low malignant potential or borderline ovarian tumor, with or without extraovarian implantations; or
- if the expression level of the proCOL11A1 protein in said sample from said subject suspected of having a malignant ovarian tumor, or in said sample from said subject diagnosed with a low malignant potential or borderline ovarian tumor, with or without extraovarian implantations, is greater than the expression level of said proCOL11A1 protein in the control sample;
or, alternatively
wherein said subject is not selected for said treatment:
- if the presence of proCOL11A1 protein is not detected in said sample from said subject suspected of having a malignant ovarian tumor, or in said sample from said subject diagnosed with a low malignant potential or borderline ovarian tumor, with or without extraovarian implantations; or
- if the expression level of the proCOL11A1 protein in said sample from said subject suspected of having a malignant ovarian tumor, or in said sample from said subject diagnosed with a low malignant potential or borderline ovarian tumor, with or without extraovarian implantations, is equal to or less than the expression level of said proCOL11A1 protein in the control sample.

5. A method for selecting a subject diagnosed with a low malignant potential or borderline ovarian tumor, with or without extraovarian implantations, for follow-up, wherein said follow-up comprises surgically resectioning said tumor and monitoring tumor recurrence, said method comprising:
- detecting the presence of proCOL11A1 protein in a sample obtained by means of biopsy, cytology or surgical resection from said subject diagnosed with a low malignant potential or borderline ovarian tumor, with or without extraovarian implantations; or
- comparing the expression level of the proCOL11A1 protein in said sample from said subject diagnosed with a low malignant potential or borderline ovarian tumor, with or without extraovarian implantations, with the expression level of said proCOL11A1 protein in a control sample;
wherein said subject is selected for said follow-up:
- if the presence of proCOL11A1 protein is detected in said sample from the subject diagnosed with a low malignant potential or borderline ovarian tumor, with or without extraovarian implantations; or
- if the expression level of the proCOL11A1 protein in said sample from said subject diagnosed with a low malignant potential or borderline ovarian tumor, with or without extraovarian implantations, is greater than the expression level of said proCOL11A1 protein in the control sample.

6. The method according to any of claims 1 to 6, which further comprises obtaining a protein extract from said sample.

7. The method according to any of claims 1 to 7, wherein the detection of the proCOL11A1 protein comprises contacting said sample, or a protein extract from said sample, with a proCOL11A1 protein-specific antibody, under conditions allowing the formation of an antibody-proCOL11A1 protein complex.

8. The method according to claim 8, further comprising the detection and/or quantification the antibody-proCOL11A1 protein complex formed, wherein the detection and/or quantification of the antibody-proCOL11A1 protein complex formed is carried out by means of a technique selected from the group consisting of Western-blot, ELISA, RIA, competitive EIA, DAS-ELISA, immunocytochemical and immunohistochemical techniques, multiplex detection techniques based on the use of protein microarrays, microspheres or biochips including specific antibodies, or assays based on colloidal precipitation.

9. The method according to any of claims 8 to 9, wherein the detection of the proCOL11A1 protein is carried out by means of an immunohistochemical analysis.

10. The method according to any of claims 4 or 5, wherein the surgical removal of said ovarian tumor is carried out by means of a method assuring complete lesion removal, comprising cytoreduction, hysterectomy, adnexectomy, colostomy and/or lymph node removal.

11. The method according to any of claims 4 or 5, wherein said chemotherapy treatment comprises the administration of an anti-tumor drug, wherein said anti-tumor drug is selected from the group consisting of paclitaxel, altretamine, capecitabine, cyclophosphamide, etoposide, gemcitabine, doxorubicin, irinotecan, topotecan, and the combinations thereof.

12. The method according to any of claims 11 to 12, wherein said treatment further comprises a radiotherapy treatment.

13. The method according to claim 4, wherein said follow-up comprises performing periodic diagnostic tests to look for relapses of the ovarian tumor or onset of metastasis.

14. Use of the proCOL11A1 protein as a marker for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

15. *In vitro* use of a specific antibody recognizing the proCOL11A1 protein for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

16. The method according to claim 8, or use of a specific antibody recognizing the proCOL11A1 protein according to claim 16, wherein said proCOL11A1 protein-specific antibody is an antibody recognizing an epitope located in the VAR domain of the N-terminal end of the proCOL11A1 protein.

17. The method according to any of claims 8 or 17, or use of a specific antibody recognizing the proCOL11A1 protein according to any of claims 16 or 17, wherein said proCOL11A1 protein-specific antibody is the monoclonal antibody 1E8.33.

18. *In vitro* use of a kit comprising a reagent recognizing the proCOL11A1 protein, or a reagent for the detection and/or quantification of the expression of the proCOL11A1 protein, for:
a) detecting a malignant ovarian tumor, wherein said malignant ovarian tumor is a malignant tumor in ovaries which can be serous, mucinous, endometrioid, clear cell tumor, or undifferentiated tumor; or for
b) performing differential diagnosis of a malignant ovarian tumor, wherein said malignant ovarian tumor is a malignant tumor in ovaries which can be serous, mucinous, endometrioid, clear cell tumor, or undifferentiated tumor, from a benign or *in situ* ovarian tumor or from a benign non-tumoral ovarian lesion; or for
c) making a diagnosis or prognosis of ovarian tumor malignancy, or for
d) determining the malignization probability of a low malignant potential or borderline ovarian tumor, with or without extraovarian implantations; or for
e) selecting a subject suspected of having a malignant ovarian tumor, wherein said malignant ovarian tumor is a malignant tumor in ovaries which can be serous, mucinous, endometrioid, clear cell tumor, or undifferentiated tumor, for treatment which comprises surgically removing the tumor, performing additional analyses to evaluate the degree of tumor invasion, applying a chemotherapy and/or radiotherapy treatment, and a subsequent follow-up; or for
f) selecting a subject diagnosed with a low malignant potential or borderline ovarian tumor, with or without extraovarian implantations, for treatment which comprises surgically removing said tumor, choosing a chemotherapy and/or radiotherapy treatment, and the subsequent follow-up; or for
g) selecting a subject suspected of having a malignant ovarian tumor, wherein said malignant ovarian tumor is a malignant tumor in ovaries which can be serous, mucinous, endometrioid, clear cell tumor, or undifferentiated tumor, wherein said follow-up comprises performing complementary analyses for assessing the degree of tumor infiltration for the purpose of determining the subsequent treatment; or for
h) selecting a subject with a low malignant potential or borderline ovarian tumor, with or without extraovarian implantations, wherein said follow-up comprises surgically resectioning the tumor and monitoring tumor recurrence; or for
i) selecting a subject with a low malignant potential or borderline ovarian tumor, with or without extraovarian implantations, wherein said follow-up comprises monitoring tumor recurrence.

## Patentansprüche

1. *In vitro*-Verfahren zur Differentialdiagnose zwischen einem malignen Eierstocktumor, wobei der maligne Eierstocktumor ein maligner Tumor in den Eierstöcken ist, der ein seröser, muzinöser, endometrioider Klarzelltumor oder undifferenzierter Tumor sein kann, und einem benignen oder *in situ*-Eierstocktumor oder einer benignen nicht-tumoralen Eierstock-Läsion in einem Individuum, ausgewählt aus Verfahren (A) und (B), wobei
A) Verfahren (A) umfasst:
Nachweisen des Vorliegens des proCOL11A1-Proteins in einer Probe, die durch Biopsie, Cytologie oder chirurgische Resektion von dem Individuum erhalten wurde, wobei
- der Nachweis des Vorliegens des proCOL11A1-Proteins in der Probe darauf hinweist, dass der Eierstocktumor maligne ist; oder alternativ
- der fehlende Nachweis des Vorliegens des proCOL11A1-Proteins in der Probe darauf hinweist, dass der Eierstocktumor benigne oder *in situ* ist oder dass das Individuum eine benigne nicht-tumorale Eierstock-Läsion aufweist; und
B) Verfahren (B) umfasst:
- Vergleichen des Expressionsspiegels des proCOL11A1-Proteins in der Probe mit dem Expressionsspiegel des proCOL11A1-Proteins in einer Kontrollprobe;
wobei
- ein Expressionsspiegel des proCOL11A1-Proteins in der Probe, der höher ist als der Expressionsspiegel des proCOL11A1-Proteins in einer Kontrollprobe, darauf hinweist, dass der Eierstocktumor ein maligner Tumor ist, oder alternativ
- ein Expressionsspiegel des proCOL11A1-Proteins in der Probe, der gleich oder niedriger ist als der Expressionsspiegel des proCOL11A1-Proteins in einer Kontrollprobe, darauf hinweist, dass der Eierstocktumor benigne oder *in situ* ist oder dass das Individuum eine benigne nicht-tumorale Eierstock-Läsion aufweist.

2. *In vitro*-Verfahren zur Diagnose oder Prognose einer Eierstocktumormalignität in einem Individuum, wobei das Individuum ein Individuum ist, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, oder ein Individuum ist, bei dem ein geringes malignes Potential oder ein Borderline-Eierstocktumor diagnostiziert wurde, mit oder ohne extraovariellen Implantaten, ausgewählt aus Verfahren (A) und Verfahren (B), wobei
A) Verfahren (A) umfasst
- Nachweisen des Vorliegens des proCOL11A1-Proteins in einer Probe, die durch Biopsie, Cytologie oder chirurgische Resektion von dem Individuum erhalten wurde, von dem angenommen wird, dass es einen malignen Eierstocktumor aufweist oder bei dem ein geringes malignes Potential oder ein Borderline-Eierstocktumor diagnostiziert wurde, mit oder ohne extraovariellen Implantaten;
wobei
- der Nachweis des Vorliegens des proCOL11A1-Proteins in der Probe des Individuums, von dem angenommen wird, dass es einen malignen Eierstocktumor aufweist oder bei dem ein geringes malignes Potential oder ein Borderline-Eierstocktumor diagnostiziert wurde, mit oder ohne extraovariellen Implantaten, darauf hinweist, dass das Individuum einen malignen Eierstocktumor aufweist oder dass das geringe maligne Potential oder der Borderline-Tumor, mit oder ohne extraovariellen Implantaten, in der Lage ist, sich in einen malignen Eierstocktumor zu entwickeln, oder alternativ,
- der fehlende Nachweis des Vorliegens des proCOL11A1-Proteins in der Probe des Individuums, von dem angenommen wird, dass es einen malignen Eierstocktumor aufweist oder bei dem ein geringes malignes Potential oder ein Borderline-Eierstocktumor diagnostiziert wurde, mit oder ohne extraovariellen Implantaten, darauf hinweist, dass das Individuum keinen malignen Eierstocktumor aufweist oder dass das Individuum einen benignen oder *in situ*-Eierstocktumor aufweist oder dass das geringe maligne Potential oder der Borderline-Tumor nicht in der Lage ist, sich in einen malignen Eierstocktumor zu entwickeln; und
B) Verfahren (B) umfasst:
- Vergleichen des Expressionsspiegels des proCOL11A1-Proteins in der Probe des Individuums, von dem angenommen wird, dass es einen malignen Eierstocktumor aufweist oder bei dem ein geringes malignes Potential oder ein Borderline-Eierstocktumor diagnostiziert wurde, mit oder ohne extraovariellen Implantaten, mit dem Expressionsspiegel des proCOL11A1-Proteins in einer Kontrollprobe;
wobei
- ein Expressionsspiegel des proCOL11A1-Proteins in der Probe des Individuums, von dem angenommen wird, dass es einen malignen Eierstocktumor aufweist oder bei dem ein geringes malignes Potential oder ein Borderline-Eierstocktumor diagnostiziert wurde, mit oder ohne extraovariellen Implantaten, der höher ist als der Expressionsspiegel des proCOL11A1-Proteins in einer Kontrollprobe, darauf hinweist, dass das Individuum einen malignen Eierstocktumor aufweist oder dass das geringe maligne Potential oder der Borderline-Tumor in der Lage ist, sich in einen malignen Eierstocktumor zu entwickeln, oder alternativ
- ein Expressionsspiegel des proCOL11A1-Proteins in der Probe des Individuums, von dem angenommen wird, dass es einen malignen Eierstocktumor aufweist oder bei dem ein geringes malignes Potential oder ein Borderline-Eierstocktumor diagnostiziert wurde, mit oder ohne extraovariellen Implantaten, der gleich oder niedriger ist als der Expressionsspiegel des proCOL11A1-Proteins in einer Kontrollprobe, darauf hinweist, dass das Individuum keinen malignen Eierstocktumor aufweist oder dass das Individuum einen benignen oder *in situ*-Eierstocktumor aufweist oder dass das geringe maligne Potential oder der Borderline-Tumor nicht in der Lage ist, sich in einen malignen Eierstocktumor zu entwickeln.

3. *In vitro*-Verfahren zur Bestimmung der Malignisierungs-Wahrscheinlichkeit eines geringen malignen Potentials oder eines Borderline-Eierstocktumors, mit oder ohne extraovariellen Implantaten, in einem Individuum, bei dem der Tumor diagnostiziert wurde, ausgewählt aus Verfahren (A) und Verfahren (B), wobei
A) Verfahren (A) umfasst
- Nachweisen des Vorliegens des proCOL11A1-Proteins in einer Probe, die durch Biopsie, Cytologie oder chirurgische Resektion von dem Individuum erhalten wurde, bei dem ein geringes malignes Potential oder ein Borderline-Eierstocktumor diagnostiziert wurde, mit oder ohne extraovariellen Implantaten;
wobei
- der Nachweis des Vorliegens des proCOL11A1-Proteins in der Probe des Individuums, bei dem ein geringes malignes Potential oder ein Borderline-Eierstocktumor diagnostiziert wurde, mit oder ohne extraovariellen Implantaten, darauf hinweist, dass die Wahrscheinlichkeit, dass der Tumor sich in einen malignen Eierstocktumor entwickelt, hoch ist, oder alternativ
- der fehlende Nachweis des Vorliegens des proCOL11A1-Proteins in der Probe des Individuums, bei dem ein geringes malignes Potential oder ein Borderline-Eierstocktumor diagnostiziert wurde, mit oder ohne extraovariellen Implantaten, darauf hinweist, dass die Wahrscheinlichkeit, dass der Tumor sich in einen malignen Eierstocktumor entwickelt, gering ist; und
B) Verfahren (B) umfasst
- Vergleichen des Expressionsspiegels des proCOL11A1-Proteins in der Probe des Individuums, bei dem ein geringes malignes Potential oder ein Borderline-Eierstocktumor diagnostiziert wurde, mit oder ohne extraovariellen Implantaten, mit dem Expressionsspiegel des proCOL11A1-Proteins in einer Kontrollprobe;
wobei
- ein Expressionsspiegel des proCOL11A1-Proteins in der Probe des Individuums, bei dem ein geringes malignes Potential oder ein Borderline-Eierstocktumor diagnostiziert wurde, mit oder ohne extraovariellen Implantaten, der höher ist als der Expressionsspiegel des proCOL11A1-Proteins in einer Kontrollprobe, darauf hinweist, dass die Wahrscheinlichkeit, dass der Tumor sich in einen malignen Eierstocktumor entwickelt, hoch ist; oder alternativ
- ein Expressionsspiegel des proCOL11A1-Proteins in der Probe des Individuums, bei dem ein geringes malignes Potential oder ein Borderline-Eierstocktumor diagnostiziert wurde, mit oder ohne extraovariellen Implantaten, der gleich oder niedriger ist als der Expressionsspiegel des proCOL11A1-Proteins in einer Kontrollprobe, darauf hinweist, dass die Wahrscheinlichkeit, dass der Tumor sich in einen malignen Eierstocktumor entwickelt, gering ist;

4. Verfahren zum Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, zur Behandlung, oder zum Auswählen eines Individuums, bei dem ein geringes malignes Potential oder ein Borderline-Tumor diagnostiziert wurde, mit oder ohne extraovariellen Implantaten, zur Behandlung, wobei die Behandlung das chirurgische Entfernen des Eierstocktumors, die Durchführung zusätzlicher Untersuchungen zur Bewertung der Tumorinfiltration und Metastasierung, wenn von dem Individuum angenommen wird, dass es einen malignen Eierstocktumor aufweist, das Auswählen einer Chemotherapie- und/oder Strahlentherapie-Behandlung, und die anschließende Nachbehandlung umfasst, wobei das Verfahren umfasst:
- Nachweisen des Vorliegens des proCOL11A1-Proteins in einer Probe, die durch Biopsie, Cytologie oder chirurgische Resektion von dem Individuum erhalten wurde, von dem angenommen wird, dass es den malignen Eierstocktumor aufweist, oder von dem Individuum, bei dem ein geringes malignes Potential oder ein Borderline-Eierstocktumor diagnostiziert wurde, mit oder ohne extraovariellen Implantaten; oder
- Vergleichen des Expressionsspiegels des proCOL11A1-Proteins in der Probe des Individuums, von dem angenommen wird, dass es einen malignen Eierstocktumor aufweist, oder in der Probe des Individuums, bei dem ein geringes malignes Potential oder ein Borderline-Eierstocktumor diagnostiziert wurde, mit oder ohne extraovariellen Implantaten, mit dem Expressionsspiegel des proCOL11A1-Proteins in einer Kontrollprobe;
wobei das Individuum für die Behandlung ausgewählt wird:
- wenn das Vorliegen des proCOL11A1-Proteins in der Probe des Individuums nachgewiesen wird, von dem angenommen wird, dass es einen malignen Eierstocktumor aufweist, oder in der Probe des Individuums, bei dem ein geringes malignes Potential oder ein Borderline-Eierstocktumor diagnostiziert wurde, mit oder ohne extraovariellen Implantaten; oder
- wenn der Expressionsspiegel des proCOL11A1-Proteins in der Probe des Individuums, von dem angenommen wird, dass es einen malignen Eierstocktumor aufweist, oder in der Probe des Individuums, bei dem ein geringes malignes Potential oder ein Borderline-Eierstocktumor diagnostiziert wurde, mit oder ohne extraovariellen Implantaten, höher ist als der Expressionsspiegel des proCOL11A1-Proteins in der Kontrollprobe;
oder alternativ
wobei das Individuum nicht für die Behandlung ausgewählt wird:
- wenn das Vorliegen des proCOL11A1-Proteins nicht in der Probe des Individuums nachgewiesen wird, von dem angenommen wird, dass es einen malignen Eierstocktumor aufweist, oder in der Probe des Individuums, bei dem ein geringes malignes Potential oder ein Borderline-Eierstocktumor diagnostiziert wurde, mit oder ohne extraovariellen Implantaten; oder
- wenn der Expressionsspiegel des proCOL11A1-Proteins in der Probe des Individuums, von dem angenommen wird, dass es einen malignen Eierstocktumor aufweist, oder in der Probe des Individuums, bei dem ein geringes malignes Potential oder ein Borderline-Eierstocktumor diagnostiziert wurde, mit oder ohne extraovariellen Implantaten, gleich oder niedriger ist als der Expressionsspiegel des proCOL11A1-Proteins in der Kontrollprobe.

5. Verfahren zum Auswählen eines Individuums, bei dem ein geringes malignes Potential oder ein Borderline-Eierstocktumor diagnostiziert wurde, mit oder ohne extraovariellen Implantaten, zur Nachbehandlung, wobei die Nachbehandlung die chirurgische Resektion des Tumors und das Überwachen von Tumorrezidiven umfasst, wobei das Verfahren umfasst:
- Nachweisen des Vorliegens des proCOL11A1-Proteins in einer Probe, die durch Biopsie, Cytologie oder chirurgische Resektion von dem Individuum erhalten wurde, bei dem ein geringes malignes Potential oder ein Borderline-Eierstocktumor diagnostiziert wurde, mit oder ohne extraovariellen Implantaten; oder
- Vergleichen des Expressionsspiegels des proCOL11A1-Proteins in der Probe des Individuums, bei dem ein geringes malignes Potential oder ein Borderline-Eierstocktumor diagnostiziert wurde, mit oder ohne extraovariellen Implantaten, mit dem Expressionsspiegel des proCOL11A1-Proteins in einer Kontrollprobe;
wobei das Individuum für die Nachbehandlung ausgewählt wird:
- wenn das Vorliegen des proCOL11A1-Proteins in der Probe des Individuums nachgewiesen wird, bei dem ein geringes malignes Potential oder ein Borderline-Eierstocktumor diagnostiziert wurde, mit oder ohne extraovariellen Implantaten; oder
- wenn der Expressionsspiegel des proCOL11A1-Proteins in der Probe des Individuums, bei dem ein geringes malignes Potential oder ein Borderline-Eierstocktumor diagnostiziert wurde, mit oder ohne extraovariellen Implantaten, höher ist als der Expressionsspiegel des proCOL11A1-Proteins in der Kontrollprobe.

6. Verfahren nach einem der Ansprüche 1 bis 6, das des Weiteren das Erhalten eines Proteinextrakts aus der Probe umfasst.

7. Verfahren nach einem der Ansprüche 1 bis 7, wobei der Nachweis des proCOL11A1-Proteins das Inkontaktbringen der Probe oder eines Proteinextrakts aus der Probe mit einem proCOL11A1-Protein-spezifischen Antikörper unter Bedingungen umfasst, die die Bildung eines Antikörper- proCOL11A1-Protein-Komplexes ermöglichen.

8. Verfahren nach Anspruch 8, das des Weiteren den Nachweis und/oder die Quantifizierung des gebildeten Antikörper-proCOL11A1-Protein-Komplexes umfasst, wobei der Nachweis und/oder die Quantifizierung des gebildeten Antikörper-proCOL11A1-Protein-Komplexes durch ein Verfahren durchgeführt wird, das ausgewählt ist aus der Gruppe bestehend aus Western-Blot, ELISA, RIA, kompetitivem EIA, DAS-ELISA, immuncytochemischen und immunhistochemischen Verfahren, Multiplex-Nachweisverfahren, die auf der Verwendung von Protein-Microarrays, Microsphären oder Biochips, die spezifische Antikörper einschließen, oder auf Assays beruhen, die auf kolloidaler Präzipitation beruhen.

9. Verfahren nach einem der Ansprüche 8 bis 9, wobei der Nachweis des proCOL1A1-Proteins durch eine immunhistochemische Analyse durchgeführt wird.

10. Verfahren nach einem der Ansprüche 4 oder 5, wobei das chirurgische Entfernen des Eierstocktumors durch ein Verfahren durchgeführt wird, das die komplette Entfernung der Läsion versichert, umfassend Zytoreduktion, Hysterektomie, Adnexektomie, Kolostomie und /oder Lymphknotenentfernung.

11. Verfahren nach einem der Ansprüche 4 oder 5, wobei die Chemotherapie-Behandlung die Verabreichung eines Anti-Tumor-Medikaments umfasst, wobei das Anti-Tumor-Medikament ausgewählt ist aus Paclitaxel, Altretamin, Capecitabin, Cyclophosphamid, Etoposid, Gemcitabin, Doxorubicin, Irinotecan, Topotecan und Kombinationen davon.

12. Verfahren nach einem der Ansprüche 11 bis 12, wobei die Behandlung des Weiteren eine Strahlentherapie-Behandlung umfasst.

13. Verfahren nach Anspruch 4, wobei die Nachbehandlung die Durchführung von periodischen diagnostischen Tests umfasst, um nach Rezidiven des Eierstocktumors oder Beginn von Metastasenbildung zu suchen.

14. Verwendung des proCOL11A1-Proteins als Marker zum:
a) Nachweisen eines malignen Eierstocktumors, wobei der maligne Eierstocktumor ein maligner Tumor in den Eierstöcken ist, der ein seröser, muzinöser, endometrioider Klarzelltumor oder undifferenzierter Tumor sein kann; oder zum
b) Durchführen einer Differentialdiagnose zwischen einem malignen Eierstocktumor, wobei der maligne Eierstocktumor ein maligner Tumor in den Eierstöcken ist, der ein seröser, muzinöser, endometrioider Klarzelltumor oder undifferenzierter Tumor sein kann, und einem benignen oder *in situ*-Eierstocktumor oder einer benignen nicht-tumoralen Eierstock-Läsion; oder zum
c) Erstellen einer Diagnose oder Prognose einer Eierstocktumormalignität, oder zum
d) Bestimmen der Malignisierungs-Wahrscheinlichkeit eines geringen malignen Potentials oder eines Borderline-Eierstocktumors, mit oder ohne extraovariellen Implantaten; oder zum
e) Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, zur Behandlung, die das chirurgische Entfernen des Tumors, die Durchführung zusätzlicher Untersuchungen zur Bewertung des Grades der Tumorinvasion, das Anwenden einer Chemotherapie- und/oder Strahlentherapie-Behandlung und eine anschließende Nachbehandlung umfasst; oder zum
f) Auswählen eines Individuums, bei dem ein geringes malignes Potential oder ein Borderline-Eierstocktumor diagnostiziert wurde, mit oder ohne extraovariellen Implantaten, zur Behandlung, die das chirurgische Entfernen des Tumors, das Auswählen einer Chemotherapie- und/oder Strahlentherapie-Behandlung und die anschließende Nachbehandlung umfasst; oder zum
g) Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, wobei die Nachbehandlung die Durchführung von Komplementäranalysen zur Bewertung des Grades der Tumorinfiltration zum Zwecke der Bestimmung der anschließenden Behandlung umfasst; oder zum
h) Auswählen eines Individuums mit einem geringen malignen Potential oder einem Borderline-Eierstocktumor, mit oder ohne extraovariellen Implantaten, wobei die Nachbehandlung die chirurgische Entfernung des Tumors und das Überwachen von Tumorrezidiven umfasst; oder zum
i) Auswählen eines Individuums mit geringem malignen Potential oder einem Borderline-Eierstocktumor, mit oder ohne extraovariellen Implantaten, wobei die Nachbehandlung das Überwachen von Tumorrezidiven umfasst.

15. *In vitro*-Verwendung eines spezifischen Antikörpers, der das proCOL11A1-Protein erkennt, zum:
a) Nachweisen eines malignen Eierstocktumors, wobei der maligne Eierstocktumor ein maligner Tumor in den Eierstöcken ist, der ein seröser, muzinöser, endometrioider Klarzelltumor oder undifferenzierter Tumor sein kann; oder zum
b) Durchführen einer Differentialdiagnose zwischen einem malignen Eierstocktumor, wobei der maligne Eierstocktumor ein maligner Tumor in den Eierstöcken ist, der ein seröser, muzinöser, endometrioider Klarzelltumor oder undifferenzierter Tumor sein kann, und einem benignen oder *in situ*-Eierstocktumor oder einer benignen nicht-tumoralen Eierstock-Läsion; oder zum
c) Erstellen einer Diagnose oder Prognose einer Eierstocktumormalignität, oder zum
d) Bestimmen der Malignisierungs-Wahrscheinlichkeit eines geringen malignen Potentials oder eines Borderline-Eierstocktumors, mit oder ohne extraovariellen Implantaten; oder zum
e) Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, zur Behandlung, die das chirurgische Entfernen des Tumors, die Durchführung zusätzlicher Untersuchungen zur Bewertung des Grades der Tumorinvasion, das Anwenden einer Chemotherapie- und/oder Strahlentherapie-Behandlung und eine anschließende Nachbehandlung umfasst; oder zum
f) Auswählen eines Individuums, bei dem ein geringes malignes Potential oder ein Borderline-Eierstocktumor diagnostiziert wurde, mit oder ohne extraovariellen Implantaten, zur Behandlung, die das chirurgische Entfernen des Tumors, das Auswählen einer Chemotherapie- und/oder Strahlentherapie-Behandlung und die anschließende Nachbehandlung umfasst; oder zum
g) Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, wobei die Nachbehandlung die Durchführung von Komplementäranalysen zur Bewertung des Grades der Tumorinfiltration zum Zwecke der Bestimmung der anschließenden Behandlung umfasst; oder zum
h) Auswählen eines Individuums mit einem geringen malignen Potential oder einem Borderline-Eierstocktumor, mit oder ohne extraovariellen Implantaten, wobei die Nachbehandlung die chirurgische Entfernung des Tumors und das Überwachen von Tumorrezidiven umfasst; oder zum
i) Auswählen eines Individuums mit geringem malignen Potential oder einem Borderline-Eierstocktumor, mit oder ohne extraovariellen Implantaten, wobei die Nachbehandlung das Überwachen von Tumorrezidiven umfasst.

16. Verfahren nach Anspruch 8 oder Verwendung eines spezifischen Antikörpers, der das proCOL11A1-Protein nach Anspruch 16 erkennt, wobei der proCOL11A1-Protein-spezifische Antikörper ein Antikörper ist, der ein Epitop erkennt, das in der VAR-Domäne des N-terminalen Endes des proCOL11A1-Proteins lokalisiert ist.

17. Verfahren nach einem der Ansprüche 8 oder 17 oder Verwendung eines spezifischen Antikörpers, der das proCOL11A1-Protein nach einem der Ansprüche 16 oder 17 erkennt, wobei der proCOL11A1-Protein-spezifische Antikörper der monoclonale Antikörper 1E8.33 ist.

18. *In vitro*-Verwendung eines Kits, umfassend ein Reagens, das das proCOL11A1-Protein erkennt, oder eines Reagens zum Nachweis und/oder zur Quantifizierung der Expression des proCOL11A1-Proteins, zum:
a) Nachweisen eines malignen Eierstocktumors, wobei der maligne Eierstocktumor ein maligner Tumor in den Eierstöcken ist, der ein seröser, muzinöser, endometrioider Klarzelltumor oder undifferenzierter Tumor sein kann; oder zum
b) Durchführen einer Differentialdiagnose zwischen einem malignen Eierstocktumor, wobei der maligne Eierstocktumor ein maligner Tumor in den Eierstöcken ist, der ein seröser, muzinöser, endometrioider Klarzelltumor oder undifferenzierter Tumor sein kann, und einem benignen oder *in situ*-Eierstocktumor oder einer benignen nicht-tumoralen Eierstock-Läsion; oder zum
c) Erstellen einer Diagnose oder Prognose einer Eierstocktumormalignität, oder zum
d) Bestimmen der Malignisierungs-Wahrscheinlichkeit eines geringen malignen Potentials oder eines Borderline-Eierstocktumors, mit oder ohne extraovariellen Implantaten; oder zum
e) Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, zur Behandlung, die das chirurgische Entfernen des Tumors, die Durchführung zusätzlicher Untersuchungen zur Bewertung des Grades der Tumorinvasion, das Anwenden einer Chemotherapie- und/oder Strahlentherapie-Behandlung und eine anschließende Nachbehandlung umfasst; oder zum
f) Auswählen eines Individuums, bei dem ein geringes malignes Potential oder ein Borderline-Eierstocktumor diagnostiziert wurde, mit oder ohne extraovariellen Implantaten, zur Behandlung, die das chirurgische Entfernen des Tumors, das Auswählen einer Chemotherapie- und/oder Strahlentherapie-Behandlung und die anschließende Nachbehandlung umfasst; oder zum
g) Auswählen eines Individuums, von dem angenommen wird, dass es einen malignen Eierstocktumor aufweist, wobei der maligne Eierstocktumor ein maligner Tumor in den Eierstöcken ist, der ein seröser, muzinöser, endometrioider Klarzelltumor oder undifferenzierter Tumor sein kann, wobei die Nachbehandlung die Durchführung von Komplementäranalysen zur Bewertung des Grades der Tumorinfiltration zum Zwecke der Bestimmung der anschließenden Behandlung umfasst; oder zum
h) Auswählen eines Individuums mit einem geringen malignen Potential oder einem Borderline-Eierstocktumor, mit oder ohne extraovariellen Implantaten, wobei die Nachbehandlung die chirurgische Entfernung des Tumors und das Überwachen von Tumorrezidiven umfasst; oder zum
i) Auswählen eines Individuums mit geringem malignen Potential oder einem Borderline-Eierstocktumor, mit oder ohne extraovariellen Implantaten, wobei die Nachbehandlung das Überwachen von Tumorrezidiven umfasst.

## Revendications

1. Procédé *in vitro* pour le diagnostic différentiel d'une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, d'une tumeur ovarienne bénigne ou *in situ* ou d'une lésion ovarienne non tumorale bénigne, chez un sujet, choisi parmi le procédé (A) et le procédé (B), où
A) le procédé (A) comprend :
la détection de la présence de la protéine proCOL11A1 dans un échantillon obtenu au moyen de biopsie, cytologie ou résection chirurgicale à partir dudit sujet où
- la détection de la présence de la protéine proCOL11A1 dans ledit échantillon indique que la tumeur ovarienne est maligne ; ou, à titre d'alternative
- la non détection de la présence de la protéine proCOL11A1 dans ledit échantillon indique que la tumeur ovarienne est bénigne ou *in situ,* ou que le sujet a une lésion ovarienne non tumorale bénigne ; et
B) le procédé (B) comprend :
- la comparaison du niveau d'expression de la protéine proCOL11A1 dans ledit échantillon avec le niveau d'expression de ladite protéine proCOL11A1 dans un échantillon témoin ;
où
- un niveau d'expression de la protéine proCOL11A1 dans ledit échantillon plus grand que le niveau d'expression de ladite protéine proCOL11A1 dans un échantillon témoin indique que la tumeur ovarienne est une tumeur maligne, ou, à titre d'alternative
- un niveau d'expression de la protéine proCOL11A1 dans ledit échantillon égal à ou plus bas que le niveau d'expression de ladite protéine proCOL11A1 dans un échantillon témoin indique que la tumeur ovarienne est bénigne ou *in situ,* ou que le sujet a une lésion ovarienne non tumorale bénigne.

2. Procédé *in vitro* pour le diagnostic ou le pronostic d'une malignité tumorale ovarienne chez un sujet, où ledit sujet est un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, ou est un sujet diagnostiqué avec une tumeur ovarienne à faible potentiel malin ou limite, avec ou sans implantations extra-ovariennes, choisi parmi le procédé (A) et le procédé (B), où
A) le procédé (A) comprend
- la détection de la présence de la protéine proCOL11A1 dans un échantillon obtenu au moyen de biopsie, cytologie ou résection chirurgicale, à partir dudit sujet suspecté d'avoir une tumeur ovarienne maligne ou diagnostiqué avec une tumeur ovarienne à faible potentiel malin ou limite, avec ou sans implantations extra-ovariennes ; où
- la détection de la présence de la protéine proCOL11A1 dans ledit échantillon provenant dudit sujet suspecté d'avoir une tumeur ovarienne maligne ou diagnostiqué avec une tumeur ovarienne à faible potentiel malin ou limite, avec ou sans implantations extra-ovariennes, indique que ledit sujet a une tumeur ovarienne maligne ou que ladite tumeur à faible potentiel malin ou limite, avec ou sans implantations extra-ovariennes, est capable d'évoluer en une tumeur ovarienne maligne, ou, à titre d'alternative
- la non-détection de la présence de la protéine proCOL11A1 dans ledit échantillon provenant dudit sujet suspecté d'avoir une tumeur ovarienne maligne ou diagnostiqué avec une tumeur ovarienne à faible potentiel malin ou limite, avec ou sans implantations extra-ovariennes, indique que ledit sujet n'a pas une tumeur ovarienne maligne, ou que ledit sujet a une tumeur ovarienne bénigne ou *in situ,* ou que ladite tumeur à faible potentiel malin ou limite n'est pas capable d'évoluer en une tumeur ovarienne maligne ; et
B) le procédé (B) comprend
- la comparaison du niveau d'expression de la protéine proCOL11A1 dans ledit échantillon provenant dudit sujet suspecté d'avoir une tumeur ovarienne maligne ou diagnostiqué avec une tumeur ovarienne à faible potentiel malin ou limite, avec ou sans implantations extra-ovariennes, avec le niveau d'expression de ladite protéine proCOL11A1 dans un échantillon témoin ;
où
- un niveau d'expression de la protéine proCOL11A1 dans ledit échantillon provenant dudit sujet suspecté d'avoir une tumeur ovarienne maligne ou diagnostiqué avec une tumeur ovarienne à faible potentiel malin ou limite, avec ou sans implantations extra-ovariennes, plus grand que le niveau d'expression de ladite protéine proCOL11A1 dans un échantillon témoin indique que ledit sujet a une tumeur ovarienne maligne ou que ledit faible potentiel malin ou ladite tumeur limite est capable d'évoluer en une tumeur ovarienne maligne, ou, à titre d'alternative
- un niveau d'expression de la protéine proCOL11A1 dans ledit échantillon provenant dudit sujet suspecté d'avoir une tumeur ovarienne maligne ou diagnostiqué avec une tumeur ovarienne à faible potentiel malin ou limite, avec ou sans implantations extra-ovariennes, égal à ou plus bas que le niveau d'expression de ladite protéine proCOL11A1 dans un échantillon témoin indique que le sujet n'a pas une tumeur ovarienne maligne, ou que ledit sujet a une tumeur ovarienne bénigne ou *in situ,* ou que ladite tumeur à faible potentiel malin ou limite n'est pas capable d'évoluer en une tumeur ovarienne maligne.

3. Procédé *in vitro* pour déterminer la probabilité de malignisation d'une tumeur ovarienne à faible potentiel malin ou limite, avec ou sans implantations extra-ovariennes, chez un sujet diagnostiqué avec ladite tumeur, choisi parmi le procédé (A) et le procédé (B), où
A) le procédé (A) comprend
- la détection de la présence de la protéine proCOL11A1 dans un échantillon obtenu au moyen de biopsie, cytologie ou résection chirurgicale à partir dudit sujet diagnostiqué avec une tumeur ovarienne à faible potentiel malin ou limite, avec ou sans implantations extra-ovariennes;
où
- la détection de la présence de la protéine proCOL11A1 dans ledit échantillon provenant dudit sujet diagnostiqué avec une tumeur ovarienne à faible potentiel malin ou limite, avec ou sans implantations extra-ovariennes, indique que la probabilité que la tumeur évolue en une tumeur ovarienne maligne est importante, ou, à titre d'alternative
- la non-détection de la présence de la protéine proCOL11A1 dans ledit échantillon provenant dudit sujet diagnostiqué avec une tumeur ovarienne à faible potentiel malin ou limite, avec ou sans implantations extra-ovariennes, indique que la probabilité que la tumeur évolue en une tumeur ovarienne maligne est faible ; et
B) le procédé (B) comprend
- la comparaison du niveau d'expression de la protéine proCOL11A1 dans ledit échantillon provenant dudit sujet diagnostiqué avec une tumeur ovarienne à faible potentiel malin ou limite, avec ou sans implantations extra-ovariennes, avec le niveau d'expression de ladite protéine proCOL11A1 dans un échantillon témoin ;
où
- - un niveau d'expression de la protéine proCOL11A1 dans ledit échantillon provenant dudit sujet diagnostiqué avec une tumeur ovarienne à faible potentiel malin ou limite, avec ou sans implantations extra-ovariennes, plus grand que le niveau d'expression de ladite protéine proCOL11A1 dans un échantillon témoin indique que la probabilité que la tumeur évolue en une tumeur ovarienne maligne est importante ; ou, à titre d'alternative
- un niveau d'expression de la protéine proCOL11A1 dans ledit échantillon provenant dudit sujet diagnostiqué avec une tumeur ovarienne à faible potentiel malin ou limite, avec ou sans implantations extra-ovariennes, égal à ou plus bas que le niveau d'expression de ladite protéine proCOL11A1 dans un échantillon témoin indique que la probabilité que la tumeur évolue en une tumeur ovarienne maligne est faible.

4. Procédé pour sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires ou une tumeur non différenciée, pour le traitement, ou pour sélectionner un sujet diagnostiqué avec une tumeur ovarienne à faible potentiel malin ou limite, avec ou sans implantations extra-ovariennes, pour le traitement, où ledit traitement comprend le retrait chirurgical de ladite tumeur ovarienne, la réalisation d'analyses supplémentaires pour évaluer l'infiltration tumorale et les métastases si le sujet est suspecté d'avoir une tumeur ovarienne maligne, le choix d'un traitement par chimiothérapie et/ou radiothérapie, et le suivi subséquent, ledit procédé comprenant :
- la détection de la présence de la protéine proCOL11A1 dans un échantillon obtenu au moyen de biopsie, cytologie ou résection chirurgicale à partir dudit sujet suspecté d'avoir la tumeur ovarienne maligne, ou à partir dudit sujet diagnostiqué avec une tumeur ovarienne à faible potentiel malin ou limite, avec ou sans implantations extra-ovariennes ; ou
- la comparaison du niveau d'expression de la protéine proCOL11A1 dans ledit échantillon provenant dudit sujet suspecté d'avoir une tumeur ovarienne maligne, ou dans ledit échantillon provenant dudit sujet diagnostiqué avec une tumeur ovarienne à faible potentiel malin ou limite, avec ou sans implantations extra-ovariennes, avec le niveau d'expression de ladite protéine proCOL11A1 dans un échantillon témoin ; où ledit sujet est sélectionné pour ledit traitement :
- si la présence de la protéine proCOL11A1 est détectée dans ledit échantillon provenant du sujet suspecté d'avoir une tumeur ovarienne maligne, ou dans ledit échantillon provenant dudit sujet diagnostiqué avec une tumeur ovarienne à faible potentiel malin ou limite, avec ou sans implantations extra-ovariennes ; ou
- si le niveau d'expression de la protéine proCOL11A1 dans ledit échantillon provenant dudit sujet suspecté d'avoir une tumeur ovarienne maligne, ou dans ledit échantillon provenant dudit sujet diagnostiqué avec une tumeur ovarienne à faible potentiel malin ou limite, avec ou sans implantations extra-ovariennes, est plus grand que le niveau d'expression de ladite protéine proCOL11A1 dans l'échantillon témoin ; ou, à titre d'alternative où ledit sujet n'est pas sélectionné pour ledit traitement :
- si la présence de la protéine proCOL11A1 n'est pas détectée dans ledit échantillon provenant dudit sujet suspecté d'avoir une tumeur ovarienne maligne, ou dans ledit échantillon provenant dudit sujet diagnostiqué avec une tumeur ovarienne à faible potentiel malin ou limite, avec ou sans implantations extra-ovariennes ; ou
- si le niveau d'expression de la protéine proCOL11A1 dans ledit échantillon provenant dudit sujet suspecté d'avoir une tumeur ovarienne maligne, ou dans ledit échantillon provenant dudit sujet diagnostiqué avec une tumeur ovarienne à faible potentiel malin ou limite, avec ou sans implantations extra-ovariennes, est égal à ou plus bas que le niveau d'expression de ladite protéine proCOL11A1 dans l'échantillon témoin.

5. Procédé pour sélectionner un sujet diagnostiqué avec une tumeur ovarienne à faible potentiel malin ou limite, avec ou sans implantations extra-ovariennes, pour le suivi, où ledit suivi comprend la résection chirurgicale de ladite tumeur et la surveillance de la récurrence de la tumeur, ledit procédé comprenant :
- la détection de la présence de la protéine proCOL11A1 dans un échantillon obtenu au moyen de biopsie, cytologie ou résection chirurgicale à partir dudit sujet diagnostiqué avec une tumeur ovarienne à faible potentiel malin ou limite, avec ou sans implantations extra-ovariennes ; ou
- la comparaison du niveau d'expression de la protéine proCOL11A1 dans ledit échantillon provenant dudit sujet diagnostiqué avec une tumeur ovarienne à faible potentiel malin ou limite, avec ou sans implantations extra-ovariennes, avec le niveau d'expression de ladite protéine proCOL11A1 dans un échantillon témoin ;
où ledit sujet est sélectionné pour ledit suivi :
- si la présence de la protéine proCOL11A1 est détectée dans ledit échantillon provenant du sujet diagnostiqué avec une tumeur ovarienne à faible potentiel malin ou limite, avec ou sans implantations extra-ovariennes ; ou
- si le niveau d'expression de la protéine proCOL11A1 dans ledit échantillon provenant dudit sujet diagnostiqué avec une tumeur ovarienne à faible potentiel malin ou limite, avec ou sans implantations extra-ovariennes, est plus grand que le niveau d'expression de ladite protéine proCOL11A1 dans l'échantillon témoin.

6. Procédé selon l'une quelconque des revendications 1 à 6, qui comprend en outre l'obtention d'un extrait de protéine à partir dudit échantillon.

7. Procédé selon l'une quelconque des revendications 1 à 7, où la détection de la protéine proCOL11A1 comprend la mise en contact dudit échantillon, ou d'un extrait de protéine provenant dudit échantillon avec un anticorps spécifique de la protéine proCOL11A1, dans des conditions permettant la formation d'un complexe anticorps-protéine proCOL11A1.

8. Procédé selon la revendication 8, comprenant en outre la détection et/ou la quantification du complexe anticorps-protéine proCOL11A1 formé, où la détection et/ou quantification du complexe anticorps-protéine proCOL11A1 formé est réalisée au moyen d'une technique choisie dans le groupe consistant en le transfert de Western, ELISA, RIA, EIA compétitif, DAS-ELISA, les techniques immunocytochimiques et immunohistochimiques, les techniques de détection multiplex basées sur l'utilisation de puces à protéines, de microsphères ou de biopuces incluant des anticorps spécifiques, ou les tests basés sur la précipitation colloïdale.

9. Procédé selon l'une quelconque des revendications 8 à 9, où la détection de la protéine proCOL11A1 est réalisée au moyen d'une analyse immunohistochimique.

10. Procédé selon l'une quelconque des revendications 4 ou 5, où le retrait chirurgical de ladite tumeur ovarienne est réalisé au moyen d'un procédé assurant le retrait complet de la lésion, comprenant la cytoréduction, l'hystérectomie, l'adnexectomie, la colostomie et/ou le retrait de ganglions lymphatiques.

11. Procédé selon l'une quelconque des revendications 4 ou 5, où ledit traitement de chimiothérapie comprend l'administration d'un médicament antitumoral, où ledit médicament antitumoral est sélectionné dans le groupe consistant en paclitaxel, altretamine, capecitabine, cyclophosphamide, étoposide, gemcitabine, doxorubicine, irinotécan, topotécan, et leurs combinaisons.

12. Procédé selon l'une quelconque des revendications 11 à 12, où ledit traitement comprend en outre un traitement par radiothérapie.

13. Procédé selon la revendication 4, où ledit suivi comprend la mise en oeuvre de tests de diagnostic périodiques pour rechercher des rechutes de la tumeur ovarienne ou l'apparition de métastases.

14. Utilisation de la protéine proCOL11A1 comme marqueur pour :
a) détecter une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée ; ou pour
b) réaliser le diagnostic différentiel d'une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, d'avec une tumeur ovarienne bénigne ou *in situ* ou d'avec une lésion ovarienne non tumorale bénigne ; ou pour
c) faire un diagnostic ou pronostic d'une malignité tumorale ovarienne, ou pour
d) déterminer la probabilité de malignisation d'une tumeur ovarienne à faible potentiel malin ou limite, avec ou sans implantations extra-ovariennes ; ou pour
e) sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, pour le traitement qui comprend le retrait chirurgical de la tumeur, la réalisation d'analyses supplémentaires pour évaluer le degré d'invasion tumorale, l'application d'un traitement par chimiothérapie et/ou radiothérapie, et un suivi subséquent ; ou pour
f) sélectionner un sujet diagnostiqué avec une tumeur ovarienne à faible potentiel malin ou limite, avec ou sans implantations extra-ovariennes, pour le traitement qui comprend le retrait chirurgical de ladite tumeur, le choix d'un traitement par chimiothérapie et/ou radiothérapie, et le suivi subséquent ; ou pour
g) sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, où ledit suivi comprend la réalisation d'analyses complémentaires pour estimer le degré d'infiltration tumorale dans le but de déterminer le traitement subséquent ; ou pour
h) sélectionner un sujet avec une tumeur ovarienne à faible potentiel malin ou limite, avec ou sans implantations extra-ovariennes, où ledit suivi comprend la résection chirurgicale de la tumeur et la surveillance de la récurrence de la tumeur ; ou pour
i) sélectionner un sujet avec une tumeur ovarienne à faible potentiel malin ou limite, avec ou sans implantations extra-ovariennes, où ledit suivi comprend la surveillance de la récurrence de la tumeur.

15. Utilisation *in vitro* d'un anticorps spécifique reconnaissant la protéine proCOL11A1 pour :
a) détecter une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée ; ou pour
b) réaliser le diagnostic différentiel d'une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, d'avec une tumeur ovarienne bénigne ou *in situ* ou d'avec une lésion ovarienne non tumorale bénigne ; ou pour
c) faire un diagnostic ou pronostic d'une malignité tumorale ovarienne, ou pour
d) déterminer la probabilité de malignisation d'une tumeur ovarienne à faible potentiel malin ou limite, avec ou sans implantations extra-ovariennes ; ou pour
e) sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, pour le traitement qui comprend le retrait chirurgical de la tumeur, la réalisation d'analyses supplémentaires pour évaluer le degré d'invasion tumorale, l'application d'un traitement par chimiothérapie et/ou radiothérapie, et un suivi subséquent ; ou pour
f) sélectionner un sujet diagnostiqué avec une tumeur ovarienne à faible potentiel malin ou limite, avec ou sans implantations extra-ovariennes, pour le traitement qui comprend le retrait chirurgical de ladite tumeur, le choix d'un traitement par chimiothérapie et/ou radiothérapie, et le suivi subséquent ; ou pour
g) sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, où ledit suivi comprend la réalisation d'analyses complémentaires pour évaluer le degré d'infiltration tumorale dans le but de déterminer le traitement subséquent ; ou pour
h) sélectionner un sujet avec une tumeur ovarienne à faible potentiel malin ou limite, avec ou sans implantations extra-ovariennes, où ledit suivi comprend la résection chirurgicale de la tumeur et la surveillance de la récurrence de la tumeur ; ou pour
i) sélectionner un sujet avec une tumeur ovarienne à faible potentiel malin ou limite, avec ou sans implantations extra-ovariennes, où ledit suivi comprend la surveillance de la récurrence de la tumeur.

16. Procédé selon la revendication 8, ou utilisation d'un anticorps spécifique reconnaissant la protéine proCOL11A1 selon la revendication 16, où ledit anticorps spécifique de la protéine proCOL11A1 est un anticorps reconnaissant un épitope situé dans le domaine VAR de l'extrémité N-terminale de la protéine proCOL11A1.

17. Procédé selon l'une quelconque des revendications 8 ou 17, ou utilisation d'un anticorps spécifique reconnaissant la protéine proCOL11A1 selon l'une quelconque des revendications 16 ou 17, où ledit anticorps spécifique de la protéine proCOL11A1 est l'anticorps monoclonal 1E8.33.

18. Utilisation *in vitro* d'un kit comprenant un réactif reconnaissant la protéine proCOL11A1 ou un réactif pour la détection et/ou la quantification de l'expression de la protéine proCOL11A1, pour :
a) détecter une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée ; ou pour
b) réaliser le diagnostic différentiel d'une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, d'avec une tumeur ovarienne bénigne ou *in situ* ou d'avec une lésion ovarienne non tumorale bénigne ; ou pour
c) faire un diagnostic ou pronostic d'une malignité tumorale ovarienne, ou pour
d) déterminer la probabilité de malignisation d'une tumeur ovarienne à faible potentiel malin ou limite, avec ou sans implantations extra-ovariennes ; ou pour
e) sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires, ou une tumeur non différenciée, pour le traitement qui comprend le retrait chirurgical de la tumeur, la réalisation d'analyses supplémentaires pour évaluer le degré d'invasion tumorale, l'application d'un traitement par chimiothérapie et/ou radiothérapie, et un suivi subséquent ; ou pour
f) sélectionner un sujet diagnostiqué avec une tumeur ovarienne à faible potentiel malin ou limite, avec ou sans implantations extra-ovariennes, pour le traitement qui comprend le retrait chirurgical de ladite tumeur, le choix d'un traitement par chimiothérapie et/ou radiothérapie, et le suivi subséquent ; ou pour
g) sélectionner un sujet suspecté d'avoir une tumeur ovarienne maligne, où ladite tumeur ovarienne maligne est une tumeur maligne dans les ovaires qui peut être séreuse, mucineuse, endométrioïde, une tumeur à cellules claires ou une tumeur non différenciée, où ledit suivi comprend la réalisation d'analyses complémentaires pour évaluer le degré d'infiltration tumorale dans le but de déterminer le traitement subséquent ; ou pour
h) sélectionner un sujet avec une tumeur ovarienne à faible potentiel malin ou limite, avec ou sans implantations extra-ovariennes, où ledit suivi comprend la résection chirurgicale de la tumeur et la surveillance de la récurrence de la tumeur ; ou pour
i) sélectionner un sujet avec une tumeur ovarienne à faible potentiel malin ou limite, avec ou sans implantations extra-ovariennes, où ledit suivi comprend la surveillance de la récurrence de la tumeur.
